# EUROPEAN PATENT APPLICATION

(11) **EP 3 025 727 A1**
(43) Date of publication of application: **01.06.2016**
(21) Application number: 15189162.9
(22) Date of filing: 30.09.2009
(51) Int. Cl.: A61K 38/43, C12N 15/113

(54) **METHODS OF TREATING LIVER DISEASE**

(30) Priority: 02.10.2008 US 102250 P
(62) Divisional of application: 09818423.7
(71) Applicant: The J. David Gladstone Institutes, San Francisco, CA 94158 (US)
(72) Inventor: OTT, Melanie, San Francisco, CA 94110 (US); HERKER, Eva, San Francisco, CA 94110 (US); FARESE, Robert V., Boston, MA 02108 (US); HARRIS, Charles, San Francisco, CA 94114 (US)
(74) Representative: Brasnett, Adrian Hugh

(57) **Abstract**

The present invention provides methods of treating liver steatosis, liver fibrosis and hepatitis C virus (HCV) infection; methods described include methods of reducing the incidence of complications associated with HCV and cirrhosis of the liver; and methods of reducing viral load, or reducing the time to viral clearance, or reducing morbidity or mortality in the clinical outcomes, in patients suffering from HCV infection.

SEQUENCE LISTING

<110> THE J. DAVID GLADSTONE INSTITUTES

<120> Methods of Treating Liver Disease

<130> AHB/FP7158140

<140>
<141> 2009-09-30

<150> 09818423.7
<151> 2009-09-30

<150> PCT/US2009/058981
<151> 2009-09-30

<150> 61/102,250
<151> 2008-10-02

<160> 19

<170> FastSEQ for Windows Version 4.0

<210> 1
<211> 488
<212> PRT
<213> Homo sapiens

<400> 1

<210> 2
<211> 388
<212> PRT
<213> Homo sapiens

<400> 2
Met Lys Thr Leu Ile Ala Ala Tyr Ser Gly Val Leu Arg Gly Glu Arg
1               5                   10                  15
Gln Ala Glu Ala Asp Arg Ser Gln Arg Ser His Gly Gly Pro Ala Leu
            20                  25                  30
Ser Arg Glu Gly Ser Gly Arg Trp Gly Thr Gly Ser Ser Ile Leu Ser
        35                  40                  45
Ala Leu Gln Asp Leu Phe Ser Val Thr Trp Leu Asn Arg Ser Lys Val
    50                  55                  60
Glu Lys Gln Leu Gln Val Ile Ser Val Leu Gln Trp Val Leu Ser Phe
65                  70                  75                  80
Leu Val Leu Gly Val Ala Cys Ser Ala Ile Leu Met Tyr Ile Phe Cys
                85                  90                  95
Thr Asp Cys Trp Leu Ile Ala Val Leu Tyr Phe Thr Trp Leu Val Phe
            100                 105                 110
Asp Trp Asn Thr Pro Lys Lys Gly Gly Arg Arg Ser Gln Trp Val Arg
        115                 120                 125
Asn Trp Ala Val Trp Arg Tyr Phe Arg Asp Tyr Phe Pro Ile Gln Leu
    130                 135                 140
Val Lys Thr His Asn Leu Leu Thr Thr Arg Asn Tyr Ile Phe Gly Tyr
145                 150                 155                 160
His Pro His Gly Ile Met Gly Leu Gly Ala Phe Cys Asn Phe Ser Thr
                165                 170                 175
Glu Ala Thr Glu Val Ser Lys Lys Phe Pro Gly Ile Arg Pro Tyr Leu
            180                 185                 190

Ala Thr Leu Ala Gly Asn Phe Arg Met Pro Val Leu Arg Glu Tyr Leu
        195                 200                 205
Met Ser Gly Gly Ile Cys Pro Val Ser Arg Asp Thr Ile Asp Tyr Leu
    210                 215                 220
Leu Ser Lys Asn Gly Ser Gly Asn Ala Ile Ile Ile Val Val Gly Gly
225                 230                 235                 240
Ala Ala Glu Ser Leu Ser Ser Met Pro Gly Lys Asn Ala Val Thr Leu
                245                 250                 255
Arg Asn Arg Lys Gly Phe Val Lys Leu Ala Leu Arg His Gly Ala Asp
            260                 265                 270
Leu Val Pro Ile Tyr Ser Phe Gly Glu Asn Glu Val Tyr Lys Gln Val
        275                 280                 285
Ile Phe Glu Glu Gly Ser Trp Gly Arg Trp Val Gln Lys Lys Phe Gln
    290                 295                 300
Lys Tyr Ile Gly Phe Ala Pro Cys Ile Phe His Gly Arg Gly Leu Phe
305                 310                 315                 320
Ser Ser Asp Thr Trp Gly Leu Val Pro Tyr Ser Lys Pro Ile Thr Thr
                325                 330                 335
Val Val Gly Glu Pro Ile Thr Ile Pro Lys Leu Glu His Pro Thr Gln
            340                 345                 350
Gln Asp Ile Asp Leu Tyr His Thr Met Tyr Met Glu Ala Leu Val Lys
        355                 360                 365
Leu Phe Asp Lys His Lys Thr Lys Phe Gly Leu Pro Glu Thr Glu Val
    370                 375                 380
Leu Glu Val Asn
385

<210> 3
<211> 550
<212> PRT
<213> Homo sapiens

<400> 3
Met Val Gly Glu Glu Lys Met Ser Leu Arg Asn Arg Leu Ser Lys Ser
 1               5                  10                  15
Arg Glu Asn Pro Glu Glu Asp Glu Asp Gln Arg Asn Pro Ala Lys Glu
            20                  25                  30
Ser Leu Glu Thr Pro Ser Asn Gly Arg Ile Asp Ile Lys Gln Leu Ile
        35                  40                  45
Ala Lys Lys Ile Lys Leu Thr Ala Glu Ala Glu Glu Leu Lys Pro Phe
    50                  55                  60
Phe Met Lys Glu Val Gly Ser His Phe Asp Asp Phe Val Thr Asn Leu
65                  70                  75                  80
Ile Glu Lys Ser Ala Ser Leu Asp Asn Gly Gly Cys Ala Leu Thr Thr
                85                  90                  95
Phe Ser Val Leu Glu Gly Glu Lys Asn Asn His Arg Ala Lys Asp Leu
            100                 105                 110
Arg Ala Pro Pro Glu Gln Gly Lys Ile Phe Ile Ala Arg Arg Ser Leu
        115                 120                 125
Leu Asp Glu Leu Leu Glu Val Asp His Ile Arg Thr Ile Tyr His Met
    130                 135                 140
Phe Ile Ala Leu Leu Ile Leu Phe Ile Leu Ser Thr Leu Val Val Asp
145                 150                 155                 160
Tyr Ile Asp Glu Gly Arg Leu Val Leu Glu Phe Ser Leu Leu Ser Tyr
                165                 170                 175
Ala Phe Gly Lys Phe Pro Thr Val Val Trp Thr Trp Trp Ile Met Phe
            180                 185                 190
Leu Ser Thr Phe Ser Val Pro Tyr Phe Leu Phe Gln His Trp Ala Thr
        195                 200                 205
Gly Tyr Ser Lys Ser Ser His Pro Leu Ile Arg Ser Leu Phe His Gly
    210                 215                 220
Phe Leu Phe Met Ile Phe Gln Ile Gly Val Leu Gly Phe Gly Pro Thr

225                 230                 235                 240
Tyr Val Val Leu Ala Tyr Thr Leu Pro Pro Ala Ser Arg Phe Ile Ile
                245                 250                 255
Ile Phe Glu Gln Ile Arg Phe Val Met Lys Ala His Ser Phe Val Arg
            260                 265                 270
Glu Asn Val Pro Arg Val Leu Asn Ser Ala Lys Glu Lys Ser Ser Thr
        275                 280                 285
Val Pro Ile Pro Thr Val Asn Gln Tyr Leu Tyr Phe Leu Phe Ala Pro
    290                 295                 300
Thr Leu Ile Tyr Arg Asp Ser Tyr Pro Arg Asn Pro Thr Val Arg Trp
305                 310                 315                 320
Gly Tyr Val Ala Met Lys Phe Ala Gln Val Phe Gly Cys Phe Phe Tyr
                325                 330                 335
Val Tyr Tyr Ile Phe Glu Arg Leu Cys Ala Pro Leu Phe Arg Asn Ile
            340                 345                 350
Lys Gln Glu Pro Phe Ser Ala Arg Val Leu Val Leu Cys Val Phe Asn
        355                 360                 365
Ser Ile Leu Pro Gly Val Leu Ile Leu Phe Leu Thr Phe Phe Ala Phe
    370                 375                 380
Leu His Cys Trp Leu Asn Ala Phe Ala Glu Met Leu Arg Phe Gly Asp
385                 390                 395                 400
Arg Met Phe Tyr Lys Asp Trp Trp Asn Ser Thr Ser Tyr Ser Asn Tyr
                405                 410                 415
Tyr Arg Thr Trp Asn Val Val Val His Asp Trp Leu Tyr Tyr Tyr Ala
            420                 425                 430
Tyr Lys Asp Phe Leu Trp Phe Phe Ser Lys Arg Phe Lys Ser Ala Ala
        435                 440                 445
Met Leu Ala Val Phe Ala Val Ser Ala Val Val His Glu Tyr Ala Leu
    450                 455                 460
Ala Val Cys Leu Ser Phe Phe Tyr Pro Val Leu Phe Val Leu Phe Met
465                 470                 475                 480
Phe Phe Gly Met Ala Phe Asn Phe Ile Val Asn Asp Ser Arg Lys Lys
                485                 490                 495
Pro Ile Trp Asn Val Leu Met Trp Thr Ser Leu Phe Leu Gly Asn Gly
            500                 505                 510
Val Leu Leu Cys Phe Tyr Ser Gln Glu Trp Tyr Ala Arg Gln His Cys
        515                 520                 525
Pro Leu Lys Asn Pro Thr Phe Leu Asp Tyr Val Arg Pro Arg Ser Trp
    530                 535                 540
Thr Cys Arg Tyr Val Phe
545                 550


<210> 4
<211> 522
<212> PRT
<213> Homo sapiens

<400> 4
Met Glu Pro Gly Gly Ala Arg Leu Arg Leu Gln Arg Thr Glu Gly Leu
1               5                   10                  15
Gly Gly Glu Arg Glu Arg Gln Pro Cys Gly Asp Gly Asn Thr Glu Thr
            20                  25                  30
His Arg Ala Pro Asp Leu Val Gln Trp Thr Arg His Met Glu Ala Val
        35                  40                  45
Lys Ala Gln Leu Leu Glu Gln Ala Gln Gly Gln Leu Arg Glu Leu Leu
    50                  55                  60
Asp Arg Ala Met Arg Glu Ala Ile Gln Ser Tyr Pro Ser Gln Asp Lys
65                  70                  75                  80
Pro Leu Pro Pro Pro Pro Pro Gly Ser Leu Ser Arg Thr Gln Glu Pro
                85                  90                  95
Ser Leu Gly Lys Gln Lys Val Phe Ile Ile Arg Lys Ser Leu Leu Asp
            100                 105                 110

Glu Leu Met Glu Val Gln His Phe Arg Thr Ile Tyr His Met Phe Ile
        115                 120                 125
Ala Gly Leu Cys Val Phe Ile Ile Ser Thr Leu Ala Ile Asp Phe Ile
    130                 135                 140
Asp Glu Gly Arg Leu Leu Leu Glu Phe Asp Leu Leu Ile Phe Ser Phe
145                 150                 155                 160
Gly Gln Leu Pro Leu Ala Leu Val Thr Trp Val Pro Met Phe Leu Ser
                165                 170                 175
Thr Leu Leu Ala Pro Tyr Gln Ala Leu Arg Leu Trp Ala Arg Gly Thr
            180                 185                 190
Trp Thr Gln Ala Thr Gly Leu Gly Cys Ala Leu Leu Ala Ala His Ala
        195                 200                 205
Val Val Leu Cys Ala Leu Pro Val His Val Ala Val Glu His Gln Leu
    210                 215                 220
Pro Pro Ala Ser Arg Cys Val Leu Val Phe Glu Gln Val Arg Phe Leu
225                 230                 235                 240
Met Lys Ser Tyr Ser Phe Leu Arg Glu Ala Val Pro Gly Thr Leu Arg
                245                 250                 255
Ala Arg Arg Gly Glu Gly Ile Gln Ala Pro Ser Phe Ser Ser Tyr Leu
            260                 265                 270
Tyr Phe Leu Phe Cys Pro Thr Leu Ile Tyr Arg Glu Thr Tyr Pro Arg
        275                 280                 285
Thr Pro Tyr Val Arg Trp Asn Tyr Val Ala Lys Asn Phe Ala Gln Ala
    290                 295                 300
Leu Gly Cys Val Leu Tyr Ala Cys Phe Ile Leu Gly Arg Leu Cys Val
305                 310                 315                 320
Pro Val Phe Ala Asn Met Ser Arg Glu Pro Phe Ser Thr Arg Ala Leu
                325                 330                 335
Val Leu Ser Ile Leu His Ala Thr Leu Pro Gly Ile Phe Met Leu Leu
            340                 345                 350
Leu Ile Phe Phe Ala Phe Leu His Cys Trp Leu Asn Ala Phe Ala Glu
        355                 360                 365
Met Leu Arg Phe Gly Asp Arg Met Phe Tyr Arg Asp Trp Trp Asn Ser
    370                 375                 380
Thr Ser Phe Ser Asn Tyr Tyr Arg Thr Trp Asn Val Val Val His Asp
385                 390                 395                 400
Trp Leu Tyr Ser Tyr Val Tyr Gln Asp Gly Leu Arg Leu Leu Gly Ala
                405                 410                 415
Arg Ala Arg Gly Val Ala Met Leu Gly Val Phe Leu Val Ser Ala Val
            420                 425                 430
Ala His Glu Tyr Ile Phe Cys Phe Val Leu Gly Phe Phe Tyr Pro Val
        435                 440                 445
Met Leu Ile Leu Phe Leu Val Ile Gly Gly Met Leu Asn Phe Met Met
    450                 455                 460
His Asp Gln Arg Thr Gly Pro Ala Trp Asn Val Leu Met Trp Thr Met
465                 470                 475                 480
Leu Phe Leu Gly Gln Gly Ile Gln Val Ser Leu Tyr Cys Gln Glu Trp
                485                 490                 495
Tyr Ala Arg Arg His Cys Pro Leu Pro Gln Ala Thr Phe Trp Gly Leu
            500                 505                 510
Val Thr Pro Arg Ser Trp Ser Cys His Thr
        515                 520

<210> 5
<211> 191
<212> PRT
<213> Hepatitis C Virus

<400> 5
Met Ser Thr Asn Pro Lys Pro Gln Arg Lys Thr Lys Arg Ser Thr Asn
 1               5                  10                  15
Arg Arg Pro Gln Asp Val Lys Phe Pro Gly Gly Gly Gln Ile Val Gly

                20                  25                  30
Gly Val Tyr Leu Leu Pro Arg Arg Gly Pro Arg Leu Gly Val Arg Ala
        35                  40                  45
Thr Arg Lys Thr Ser Glu Arg Ser Gln Pro Arg Gly Arg Arg Gln Pro
    50                  55                  60
Ile Pro Lys Ala Arg Gln Pro Glu Gly Arg Ala Trp Ala Gln Pro Gly
65                  70                  75                  80
Tyr Pro Trp Pro Leu Tyr Gly Asn Glu Gly Met Gly Trp Ala Gly Trp
                85                  90                  95
Leu Leu Ser Pro Arg Gly Ser Arg Pro Ser Trp Gly Pro Thr Asp Pro
                100                 105                 110
Arg Arg Arg Ser Arg Asn Leu Gly Lys Val Ile Asp Thr Leu Thr Cys
        115                 120                 125
Gly Phe Ala Asp Leu Met Gly Tyr Ile Pro Leu Val Gly Ala Pro Leu
    130                 135                 140
Gly Gly Ala Ala Arg Ala Leu Ala His Gly Val Arg Val Leu Glu Asp
145                 150                 155                 160
Gly Val Asn Tyr Ala Thr Gly Asn Leu Pro Gly Cys Ser Phe Ser Ile
                165                 170                 175
Phe Leu Leu Ala Leu Leu Ser Cys Leu Thr Ile Pro Ala Ser Ala
                180                 185                 190

<210> 6
<211> 1467
<212> DNA
<213> Homo sapiens

<400> 6
atgggcgacc gcggcagctc ccggcgccgg aggacagggt cgcggccctc gagccacggc 60
ggcggcgggc ctgcggcggc ggaagaggag gtgcgggacg ccgctgcggg ccccgacgtg 120
ggagccgcgg gggacgcgcc agccccggcc cccaacaagg acggagacgc cggcgtgggc 180
agcggccact gggagctgag gtgccatcgc ctgcaggatt ctttattcag ctctgacagt 240
ggcttcagca actaccgtgg catcctgaac tggtgtgtgg tgatgctgat cttgagcaat 300
gcccggttat ttctggagaa cctcatcaag tatggcatcc tggtggaccc catccaggtg 360
gtttctctgt tcctgaagga tccctatagc tggcccgccc catgcctggt tattgcggcc 420
aatgtctttg ctgtggctgc attccaggtt gagaagcgcc tggcggtggg tgccctgacg 480
gagcaggcgg gactgctgct gcacgtggcc aacctggcca ccattctgtg tttcccagcg 540
gctgtggtct tactggttga gtctatcact ccagtgggct ccctgctggc gctgatggcg 600
cacaccatcc tcttcctcaa gctcttctcc taccgcgacg tcaactcatg gtgccgcagg 660
gccagggcca aggctgcctc tgcagggaag aaggccagca gtgctgctgc cccgcacacc 720
gtgagctacc cggacaatct gacctaccgc gatctctact acttcctctt cgcccccacc 780
ttgtgctacg agctcaactt tccccgctct ccccgcatcc ggaagcgctt tctgctgcga 840
cggatccttg agatgctgtt cttcacccag ctccaggtgg ggctgatcca gcagtggatg 900
gtccccacca tccagaactc catgaagccc ttcaaggaca tggactactc acgcatcatc 960
gagcgcctcc tgaagctggc ggtccccaat cacctcatct ggctcatctt cttctactgg 1020
ctcttccact cctgcctgaa tgccgtggct gagctcatgc agtttggaga ccgggagttc 1080
taccgggact ggtggaactc cgagtctgtc acctacttct ggcagaactg gaacatccct 1140
gtgcacaagt ggtgcatcag acacttctac aagcccatgc ttcgacgggg cagcagcaag 1200
tggatggcca ggacaggggt gttcctggcc tcggccttct tccacgagta cctggtgagc 1260
gtccctctgc gaatgttccg cctctgggcg ttcacgggca tgatggctca gatcccactg 1320
gcctggttcg tgggccgctt tttccagggc aactatggca acgcagctgt gtggctgtcg 1380
ctcatcatcg gacagccaat agccgtcctc atgtacgtcc acgactacta cgtgctcaac 1440
tatgaggccc cagcggcaga ggcctga                                    1467

<210> 7
<211> 20
<212> RNA
<213> Artificial Sequence

<220>
<223> Synthetic Oligonucleotide

<400> 7
gcccauggcc ucagcccgca                                                    20

<210> 8
<211> 20
<212> DNA
<213> Artificial Sequence

<220>
<223> Synthetic Oligonucleotide

<400> 8
acgccggcgu cuccguccuu                                                    20

<210> 9
<211> 20
<212> RNA
<213> Artificial Sequence

<220>
<223> Synthetic Oligonucleotide

<400> 9
cugcaggcga uggcaccuca                                                    20

<210> 10
<211> 20
<212> RNA
<213> Artificial Sequence

<220>
<223> Synthetic Oligonucleotide

<400> 10
cucccagcug gcaucagacu                                                    20

<210> 11
<211> 19
<212> RNA
<213> Artificial Sequence

<220>
<223> Synthetic Oligonucleotide

<400> 11
cuugagcaau gcccgguua                                                     19

<210> 12
<211> 19
<212> RNA
<213> Artificial Sequence

<220>
<223> Synthetic Oligonucleotide

<400> 12
caauagccgu ccucaugua                                                     19

<210> 13
<211> 19
<212> RNA
<213> Artificial Sequence

<220>
<223> Synthetic Oligonucleotide

<400> 13
ucaaggacau ggacuacuc                                                    19

<210> 14
<211> 19
<212> RNA
<213> Artificial Sequence

<220>
<223> Synthetic Oligonucleotide

<400> 14
gcuguggucu uacugguug                                                    19

<210> 15
<211> 19
<212> RNA
<213> Artificial Sequence

<220>
<223> Synthetic Oligonucleotide

<400> 15
gaacacaccc aagaaaggu                                                    19

<210> 16
<211> 19
<212> DNA
<213> Artificial Sequence

<220>
<223> Synthetic Oligonucleotide

<400> 16
ggaacatccc tgtgcacaa                                                    19

<210> 17
<211> 19
<212> DNA
<213> Artificial Sequence

<220>
<223> Synthetic Oligonucleotide


<400> 17
gcatcagaca cttctacaa                                                    19

<210> 18
<211> 19
<212> DNA
<213> Artificial Sequence

<220>
<223> Synthetic Oligonucleotide

<400> 18
gcgaaagcca cttctcata                                                    19

<210> 19

<211> 19
<212> DNA
<213> Artificial Sequence

<220>
<223> Synthetic Oligonucleotide

<400> 19
cttacgctga gtacttcga 19

1

## Description

### CROSS-REFERENCE

This application claims the benefit of U.S. Provisional Patent Application No. 61/102,250, filed October 2, 2008, which application is incorporated herein by reference in its entirety.

### STATEMENT REGARDING FEDERALLY SPONSORED RESEARCH

The U.S. government has certain rights in this invention, pursuant to grant nos. DK056084 and AI069090 awarded by the National Institutes of Health.

### BACKGROUND

Hepatitis C virus (HCV) infection is the most common chronic blood borne infection in the United States. Although the numbers of new infections have declined, the burden of chronic infection is substantial, with Centers for Disease Control estimates of 3.9 million (1.8%) infected persons in the United States. Chronic liver disease is the tenth leading cause of death among adults in the United States, and accounts for approximately 25,000 deaths annually, or approximately 1% of all deaths. Studies indicate that 40% of chronic liver disease is HCV-related, resulting in an estimated 8,000-10,000 deaths each year. HCV-associated end-stage liver disease is the most frequent indication for liver transplantation among adults.

Antiviral therapy of chronic hepatitis C has evolved rapidly over the last decade, with significant improvements seen in the efficacy of treatment. Nevertheless, even with combination therapy using pegylated IFN-α plus ribavirin, 40% to 50% of patients fail therapy, i.e., 40% to 50% of patients are nonresponders or relapsers. These patients currently have no effective therapeutic alternative. In particular, patients who have advanced fibrosis or cirrhosis on liver biopsy are at significant risk of developing complications of advanced liver disease, including ascites, jaundice, variceal bleeding, encephalopathy, and progressive liver failure, as well as a markedly increased risk of hepatocellular carcinoma.

### Literature

U.S. Patent Publication No. 2005/0272680

### SUMMARY OF THE INVENTION

The present disclosure provides methods of treating hepatitis C virus (HCV) infection; methods of reducing the incidence of complications associated with HCV and cirrhosis of the liver; and methods of reducing viral load, or reducing the time to viral clearance, or reducing morbidity or mortality in the clinical outcomes, in patients suffering from HCV infection. Also provided are methods of treating liver steatosis and liver fibrosis.

### BRIEF DESCRIPTION OF THE DRAWINGS

Figures 1A-I depict the effect of DGAT1 on HCV core-induced lipid droplet accumulation.
Figures 2A-E depict the effect of HCV core expression on triglyceride breakdown.
Figures 3A-G depict interaction of HCV Core with DGAT1.
Figures 4A-I depict the effect of DGAT1 inhibition on HCV virion assembly.
Figures 5A-C depict the effect of lack of DGAT1 on spread of HCV infection.
Figures 6A-E depict the effect of DGAT1 inhibition on Core-mediated recruitment of viral protein and viral RNA to lipid droplets.
Figure 7 depicts an amino acid sequence of DGAT1 (SEQ ID NO:1).
Figure 8 depicts an amino acid sequence of DGAT2 (SEQ ID NO:2).
Figure 9 depicts an amino acid sequence of ACAT1 (SEQ ID NO:3).
Figure 10 depicts an amino acid sequence of ACAT2 (SEQ ID NO:4).
Figure 11 depicts an amino acid sequence of an HCV nucleocapsid (SEQ ID NO:5).
Figure 12 depicts a nucleotide sequence encoding a DGAT1 polypeptide (SEQ ID NO:6).

### DEFINITIONS

As used herein, the term "flavivirus" includes any member of the family Flaviviridae, including, but not limited to, Dengue virus, including Dengue virus 1, Dengue virus 2, Dengue virus 3, Dengue virus 4 (see, e.g., GenBank Accession Nos. M23027, M19197, A34774, and M14931); Yellow Fever Virus; West Nile Virus; Japanese Encephalitis Virus; St. Louis Encephalitis Virus; Bovine Viral Diarrhea Virus (BVDV); and Hepatitis C Virus (HCV); and any serotype, strain, genotype, subtype, quasispecies, or isolate of any of the foregoing. Where the flavivirus is HCV, the term "HCV" encompasses any of a number of genotypes, subtypes, or quasispecies, of HCV, including, e.g., genotype 1, including 1a and 1b, 2, 3, 4, 6, etc. and subtypes (e.g., 2a, 2b, 3a, 4a, 4c, etc.), and quasispecies.

As used herein, the term "hepatic fibrosis," used interchangeably herein with "liver fibrosis," refers to the growth of scar tissue in the liver that can occur in the context of a chronic hepatitis infection.

The terms "individual," "host," "subject," and "patient" are used interchangeably herein, and refer to a mammal, including, but not limited to, non-human primates (e.g., simians), and humans.

As used herein, the term "liver function" refers to a normal function of the liver, including, but not limited to, a synthetic function, including, but not limited to, synthesis of proteins such as serum proteins (e.g., albumin, clotting factors, alkaline phosphatase, aminotransferases (e.g., alanine transaminase, aspartate transaminase), 5'-nucleosidase, γ-glutaminyltranspeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics; and the like.

The term "sustained viral response" (SVR; also referred to as a "sustained response" or a "durable response"), as used herein, refers to the response of an individual to a treatment regimen for HCV infection, in terms of serum HCV titer. Generally, a "sustained viral response" refers to no detectable HCV RNA (e.g., less than about 500, less than about 200, or less than about 100 genome copies per milliliter serum) found in the patient's serum for a period of at least about one month, at least about two months, at least about three months, at least about four months, at least about five months, or at least about six months following cessation of treatment.

"Treatment failure patients" as used herein generally refers to HCV-infected patients who failed to respond to previous therapy for HCV (referred to as "non-responders") or who initially responded to previous therapy, but in whom the therapeutic response was not maintained (referred to as "relapsers").

"Treatment," as used herein, covers any treatment of a disease in a mammal, particularly in a human, and includes: (a) preventing the disease from occurring in a subject which may be predisposed to the disease but has not yet been diagnosed as having it; (b) inhibiting the disease, i.e., arresting its development; and (c) relieving the disease, i.e., causing regression of the disease.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a," "an," and "the" include plural referents unless the context clearly dictates otherwise. Thus, for example, reference to "a diacylglycerol acyltransferase-1 polypeptide" includes a plurality of such polypeptides and reference to "the lipid synthesis acyltransferase inhibitor" includes reference to one or more lipid synthesis acyltransferase inhibitors and equivalents thereof known to those skilled in the art, and so forth. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

### DETAILED DESCRIPTION

The present disclosure provides methods of treating hepatitis C virus (HCV) infection; methods of reducing the incidence of complications associated with HCV and cirrhosis of the liver; and methods of reducing viral load, or reducing the time to viral clearance, or reducing morbidity or mortality in the clinical outcomes, in patients suffering from HCV infection. Also provided are methods of treating liver steatosis and liver fibrosis.

### TREATMENT METHODS

The present disclosure provides methods of treating an HCV infection; and methods of treating complications or sequelae of an HCV infection, e.g., liver fibrosis. The methods generally involve administering to an individual in need thereof an effective amount of an active agent that reduces the level and/or activity of a lipid synthesis acyltransferase.

### Hepatitis C Virus infection

The HCV core protein localizes to the surface of lipid droplets and recruits the viral replication machinery to its proximity. HCV core interacts with lipid synthesis acyltransferase (e.g., DGAT1) at endoplasmic reticulum membranes; core gets loaded on newly synthesized lipid droplets. HCV core (also referred to herein simply as "core") at the lipid droplets recruits HCV RNA replication and assembly complexes. Inhibitors of lipid synthesis acyltransferases (e.g., DGAT1, DGAT2, ACAT1, ACAT2) can block loading of HCV core on lipid droplets, and can interfere with the assembly step of HCV.

A lipid synthesis acyltransferase inhibitor reduces the number of HCV virions produced by an HCV-infected cell. For example, in some embodiments, contacting an HCV-infected cell with a lipid synthesis acyltransferase inhibitor reduces the number of HCV virions produced by the HCV-infected cell by at least about 10%, at least about 15%, at least about 20%, at least about 25%, at least about 30%, at least about 35%, at least about 40%, at least about 45%, at least about 50%, at least about 60%, at least about 70%, at least about 80%, or at least about 90, or more than 90%, compared to the number of HCV virions produced by the HCV-infected cell not contacted with the lipid synthesis acyltransferase.

In some embodiments, an effective amount of a lipid synthesis acyltransferase inhibitor is an amount that, when administered alone (e.g., in monotherapy) in one or more doses, is effective to reduce viral load or achieve a sustained viral response to therapy. In some embodiments, an effective amount of a lipid synthesis acyltransferase inhibitor is an amount that, when administered alone (e.g., in monotherapy) in multiple (e.g., two or more) doses, is effective to reduce viral load or achieve a sustained viral response to therapy. In some embodiments, an effective amount of a lipid synthesis acyltransferase inhibitor is an amount that, when administered in one or more doses in combination therapy with at least one additional therapeutic agent, is effective to reduce viral load or achieve a sustained viral response to therapy. Suitable lipid synthesis acyltransferase inhibitors include active agents that reduce an enzymatic activity and/or a level of a lipid synthesis acyltransferase polypeptide in a cell.

Whether a subject method is effective in treating an HCV infection can be determined by measuring viral load, or by measuring a parameter associated with HCV infection, including, but not limited to, liver fibrosis, elevations in serum transaminase levels, and necroinflammatory activity in the liver. Indicators of liver fibrosis are discussed in detail below.

In some embodiments, an effective amount of a lipid synthesis acyltransferase inhibitor is an amount that, when administered to an individual in need thereof in one or more doses, or alone or in combination therapy, is effective to reduce HCV viral titers to undetectable levels, e.g., to about 1000 to about 5000, to about 500 to about 1000, or to about 100 to about 500 genome copies/mL serum. In some embodiments, an effective amount of a lipid synthesis acyltransferase inhibitor, and optionally one or more additional antiviral agents, is an amount that is effective to reduce viral load to lower than 5000 genome copies/mL serum. In some embodiments, an effective amount of a lipid synthesis acyltransferase inhibitor, and optionally one or more additional antiviral agents, is an amount that is effective to reduce viral load to lower than 1000 genome copies/mL serum. In some embodiments, an effective amount of a lipid synthesis acyltransferase inhibitor, and optionally one or more additional antiviral agents, is an amount that is effective to reduce viral load to lower than 500 genome copies/mL serum. In some embodiments, an effective amount of a lipid synthesis acyltransferase inhibitor, and optionally one or more additional antiviral agents, is an amount that is effective to reduce viral load to lower than 100 genome copies/mL serum.

In some embodiments, an effective amount of a lipid synthesis acyltransferase inhibitor is an amount that, when administered to an individual in need thereof in one or more doses, or alone or in combination therapy, is effective to achieve a 1.5-log, a 2-log, a 2.5-log, a 3-log, a 3.5-log, a 4-log, a 4.5-log, or a 5-log reduction in HCV viral titer in the serum of the individual.

In some embodiments, an effective amount of a lipid synthesis acyltransferase inhibitor is an amount that, when administered to an individual in need thereof in one or more doses, or alone or in combination therapy, is effective to achieve a sustained viral response, e.g., non-detectable or substantially non-detectable HCV RNA (e.g., less than about 500, less than about 400, less than about 200, or less than about 100 genome copies per milliliter serum) is found in the patient's serum for a period of at least about one month, at least about two months, at least about three months, at least about four months, at least about five months, or at least about six months following cessation of therapy.

As noted above, whether a subject method is effective in treating an HCV infection can be determined by measuring a parameter associated with HCV infection, such as liver fibrosis. Methods of determining the extent of liver fibrosis are discussed in detail below. In some embodiments, the level of a serum marker of liver fibrosis indicates the degree of liver fibrosis.

As one non-limiting example, levels of serum alanine aminotransferase (ALT) are measured, using standard assays. In general, an ALT level of less than about 45 international units is considered normal. In some embodiments, an effective amount of a compound of formula I, and optionally one or more additional antiviral agents, is an amount effective to reduce ALT levels to less than about 45 IU/ml serum.

In some embodiments, an effective amount of a lipid synthesis acyltransferase inhibitor is an amount that, when administered to an individual in need thereof in one or more doses, or alone or in combination therapy, is effective to reduce a serum level of a marker of liver fibrosis by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the level of the marker in an untreated individual, or to a placebo-treated individual. Methods of measuring serum markers include immunological-based methods, e.g., enzyme-linked immunosorbent assays (ELISA), radioimmunoassays, and the like, using antibody specific for a given serum marker.

Suitable lipid synthesis acyltransferase inhibitors include, but are not limited to, small molecule agents, antibodies specific for a lipid synthesis acyltransferase, and an interfering RNA that specifically reduces production of a lipid synthesis acyltransferase.

In some embodiments, an active agent (a lipid synthesis acyltransferase inhibitor) reduces enzymatic activity of a lipid synthesis acyltransferase by at least about 10%, at least about 15%, at least about 20%, at least about 25%, at least about 30%, at least about 35%, at least about 40%, at least about 45%, at least about 50%, at least about 60%, at least about 70%, or at least about 80%, or more, compared to the enzymatic activity of the lipid synthesis acyltransferase in the absence of the inhibitor. Small molecule agents are examples of active agents that can reduce enzymatic activity of a lipid synthesis acyltransferase.

In some embodiments, an active agent (a lipid synthesis acyltransferase inhibitor) reduces interaction between a lipid synthesis acyltransferase and an HCV core protein. For example, in some embodiments, an active agent reduces interaction (e.g., binding) between a lipid synthesis acyltransferase and an HCV core protein by at least about 10%, at least about 15%, at least about 20%, at least about 25%, at least about 30%, at least about 35%, at least about 40%, at least about 45%, at least about 50%, at least about 60%, at least about 70%, or at least about 80%, or more, compared to the binding of the lipid synthesis acyltransferase to the HCV core protein in the absence of the active agent. Small molecule agents and antibodies are examples of active agents that can reduce binding of an HCV core protein to a lipid synthesis acyltransferase.

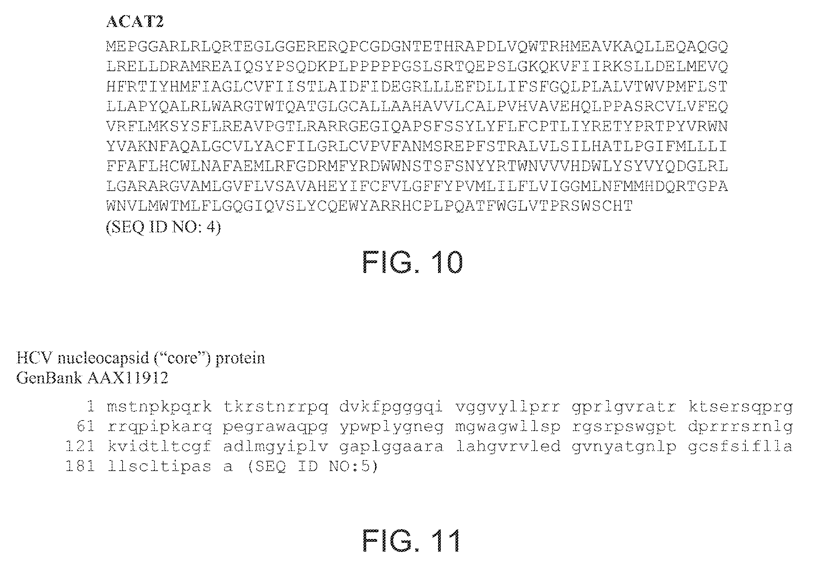

"HCV core protein" refers to the nucleocapsid protein of any serotype, strain, genotype, subtype, quasispecies, or isolate of HCV. For example, an HCV core protein can be from about 180 amino acids to about 200 amino acids in length, and can have an amino acid sequence having at least about 75%, at least about 80%, at least about 85%, at least about 90%, at least about 95%, at least about 98%, at least about 99%, or 100%, amino acid sequence identity to the amino acid sequence set forth in GenBank Accession No. AAX11912, and depicted in Figure 11 (SEQ ID NO:5).

In some embodiments, an active agent reduces the level of lipid synthesis acyltransferase activity in a cell by reducing the level of lipid synthesis acyltransferase polypeptide in the cell. For example, in some embodiments, an active agent reduces the level of lipid synthesis acyltransferase polypeptide in a cell by at least about 10%, at least about 15%, at least about 20%, at least about 25%, at least about 30%, at least about 35%, at least about 40%, at least about 45%, at least about 50%, at least about 60%, at least about 70%, or at least about 80%, or more, compared to the level of the lipid synthesis acyltransferase polypeptide in the cell in the absence of the active agent. An interfering RNA specific for a lipid synthesis acyltransferase is an example of an active agent that can reduce the level of lipid synthesis acyltransferase polypeptide in a cell.

Lipid synthesis acyltransferases include diacylglycerol acyltransferase-1 (DGAT1), diacylglycerol acyltransferase-2 (DGAT2), acyl-CoA:cholesterol acyltransferase-1 (ACAT1), and acyl-CoA:cholesterol acyltransferase-2 (ACAT2). In some embodiments, an active agent suitable for use in a subject method specifically reduces the enzymatic activity and/or level of a DGAT1 polypeptide, a DGAT2 polypeptide, an ACAT1 polypeptide, or an ACAT2 polypeptide. In other embodiments, an active agent suitable for use in a subject method reduces the enzymatic activity and/or level of two or more of a DGAT1 polypeptide, a DGAT2 polypeptide, an ACAT1 polypeptide, or an ACAT2 polypeptide.

### Liver steatosis

The present disclosure provides methods for treating hepatocellular damage resulting from HCV infection, where hepatocellular damage includes, e.g., liver steatosis, including non-alcoholic fatty liver disease. Fatty liver is defined as an excessive accumulation of triglyceride inside the liver cells. In certain embodiments, in patients with non-alcoholic fatty liver disease, liver contains more that about 5% of the total weight of the liver or more than 30% of liver cells in a liver lobule are with fat deposit. The present disclosure provides methods of treating liver steatosis in an individual, the methods generally involving administering to the individual an effective amount of an agent that reduces the level and/or enzymatic activity of a lipid synthesis acyltransferase.

In some embodiments, an "effective amounts" of an active agent (an agent that reduces the level and/or enzymatic activity of a lipid synthesis acyltransferase) is an amount that, when administered in one or more doses, in monotherapy or combination therapy, is effective to reduce the percent by weight of fat in the liver of the individual being treated by at least about 5%, at least about 10%, at least about 20%, at least about 30%, at least about 40%, at least about 50%, or more, compared with an untreated individual or a placebo-treated individual. In some embodiments, an "effective amounts" of an active agent (an agent that reduces the level and/or enzymatic activity of a lipid synthesis acyltransferase) is an amount that, when administered in one or more doses, in monotherapy or combination therapy, is effective to reduce the percent by weight of fat in the liver of the individual being treated to within a normal range.

### Liver fibrosis

Liver fibrosis is a precursor to the complications associated with liver cirrhosis, such as portal hypertension, progressive liver insufficiency, and hepatocellular carcinoma. The present disclosure provides methods of treating liver fibrosis in an individual, the methods generally involving administering to the individual an effective amount of an agent that reduces the level and/or enzymatic activity of a lipid synthesis acyltransferase. A reduction in liver fibrosis thus reduces the incidence of such complications. Accordingly, the present disclosure further provides methods of reducing the likelihood that an individual will develop complications associated with cirrhosis of the liver, the methods generally involving administering to the individual an effective amount of an agent that reduces the level and/or enzymatic activity of a lipid synthesis acyltransferase.

A therapeutically effective amount of an active agent that is administered as part of a subject treatment method is an amount that is effective to reduce a serum level of a marker of liver fibrosis by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the level of the marker in an untreated individual, or to a placebo-treated individual. Methods of measuring serum markers include immunological-based methods, e.g., ELISA, radioimmunoassays, and the like, using antibody specific for a given serum marker.

In the context of treating liver fibrosis, an "effective amounts" of an active agent (an agent that reduces the level and/or enzymatic activity of a lipid synthesis acyltransferase) is an amount that, when administered in one or more doses, in monotherapy or combination therapy, is effective in reducing liver fibrosis or reduce the rate of progression of liver fibrosis; and/or that is effective in reducing the likelihood that an individual will develop liver fibrosis; and/or that is effective in reducing a parameter associated with liver fibrosis; and/or that is effective in reducing a disorder associated with cirrhosis of the liver.

The present disclosure also provides a method for treatment of liver fibrosis in an individual comprising administering to the individual an mount of an active agent (an agent that reduces the level and/or enzymatic activity of a lipid synthesis acyltransferase) that is effective for prophylaxis or therapy of liver fibrosis in the individual, e.g., increasing the probability of survival, reducing the risk of death, ameliorating the disease burden or slowing the progression of disease in the individual.

Whether a subject treatment method is effective in reducing liver fibrosis can be determined by any of a number of well-established techniques for measuring liver fibrosis and liver function. Whether liver fibrosis is reduced is determined by analyzing a liver biopsy sample. An analysis of a liver biopsy comprises assessments of two major components: necroinflammation assessed by "grade" as a measure of the severity and ongoing disease activity, and the lesions of fibrosis and parenchymal or vascular remodeling as assessed by "stage" as being reflective of long-term disease progression. See, e.g., Brunt (2000) Hepatol. 31:241-246; and METAVIR (1994) Hepatology 20:15-20. Based on analysis of the liver biopsy, a score is assigned. A number of standardized scoring systems exist which provide a quantitative assessment of the degree and severity of fibrosis. These include the METAVIR, Knodell, Scheuer, Ludwig, and Ishak scoring systems. These methods are described in more detail below.

In some embodiments, an effective amount of an active agent (an agent that reduces the level and/or enzymatic activity of a lipid synthesis acyltransferase) is an amount that is effective to increase an index of liver function by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the index of liver function in an untreated individual, or in a placebo-treated individual. Those skilled in the art can readily measure such indices of liver function, using standard assay methods, many of which are commercially available, and are used routinely in clinical settings.

Serum markers of liver fibrosis can also be measured as an indication of the efficacy of a subject treatment method. Serum markers of liver fibrosis include, but are not limited to, hyaluronate, N-terminal procollagen III peptide, 7S domain of type IV collagen, C-terminal procollagen I peptide, and laminin. Additional biochemical markers of liver fibrosis include α-2-macroglobulin, haptoglobin, gamma globulin, apolipoprotein A, and gamma glutamyl transpeptidase.

In some embodiments, an effective amount of an active agent (an agent that reduces the level and/or enzymatic activity of a lipid synthesis acyltransferase) is an amount that is effective to reduce a serum level of a marker of liver fibrosis by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to the level of the marker in an untreated individual, or in a placebo-treated individual. Those skilled in the art can readily measure such serum markers of liver fibrosis, using standard assay methods, many of which are commercially available, and are used routinely in clinical settings. Methods of measuring serum markers include immunological-based methods, e.g., enzyme-linked immunosorbent assays (ELISA), radioimmunoassays, and the like, using antibody specific for a given serum marker.

Quantitative tests of functional liver reserve can also be used to assess the efficacy of a subject treatment. These include: indocyanine green clearance (ICG), galactose elimination capacity (GEC), aminopyrine breath test (ABT), antipyrine clearance, monoethylglycine-xylidide (MEG-X) clearance, and caffeine clearance.

As used herein, a "complication associated with cirrhosis of the liver" refers to a disorder that is a sequelae of decompensated liver disease, i.e., or occurs subsequently to and as a result of development of liver fibrosis, and includes, but is not limited to, development of ascites, variceal bleeding, portal hypertension, jaundice, progressive liver insufficiency, encephalopathy, hepatocellular carcinoma, liver failure requiring liver transplantation, and liver-related mortality.

In some embodiments, an effective amount of an active agent (an agent that reduces the level and/or enzymatic activity of a lipid synthesis acyltransferase) is an amount that is effective in reducing the incidence of (e.g., the likelihood that an individual will develop) a disorder associated with cirrhosis of the liver by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, or at least about 80%, or more, compared to an untreated individual, or in a placebo-treated individual.

Whether a subject treatment method is effective in reducing the incidence of a disorder associated with cirrhosis of the liver can readily be determined by those skilled in the art.

Reduction in liver fibrosis increases liver function. Thus, the present disclosure provides methods for increasing liver function, the method generally involving administering to an individual in need thereof an effective amount of an active agent (an agent that reduces the level and/or enzymatic activity of a lipid synthesis acyltransferase). Liver functions include, but are not limited to, synthesis of proteins such as serum proteins (e.g., albumin, clotting factors, alkaline phosphatase, aminotransferases (e.g., alanine transaminase, aspartate transaminase), 5'-nucleosidase, γ-glutaminyltranspeptidase, etc.), synthesis of bilirubin, synthesis of cholesterol, and synthesis of bile acids; a liver metabolic function, including, but not limited to, carbohydrate metabolism, amino acid and ammonia metabolism, hormone metabolism, and lipid metabolism; detoxification of exogenous drugs; a hemodynamic function, including splanchnic and portal hemodynamics; and the like.

Whether a liver function is increased is readily ascertainable by those skilled in the art, using well-established tests of liver function. Thus, synthesis of markers of liver function such as albumin, alkaline phosphatase, alanine transaminase, aspartate transaminase, bilirubin, and the like, can be assessed by measuring the level of these markers in the serum, using standard immunological and enzymatic assays. Splanchnic circulation and portal hemodynamics can be measured by portal wedge pressure and/or resistance using standard methods. Metabolic functions can be measured by measuring the level of ammonia in the serum.

Whether serum proteins normally secreted by the liver are in the normal range can be determined by measuring the levels of such proteins, using standard immunological and enzymatic assays. Those skilled in the art know the normal ranges for such serum proteins. The following are non-limiting examples. The normal range of alanine transaminase is from about 7 to about 56 units per liter of serum. The normal range of aspartate transaminase is from about 5 to about 40 units per liter of serum. Bilirubin is measured using standard assays. Normal bilirubin levels are usually less than about 1.2 mg/dL. Serum albumin levels are measured using standard assays. Normal levels of serum albumin are in the range of from about 35 to about 55 g/L. Prolongation of prothrombin time is measured using standard assays. Normal prothrombin time is less than about 4 seconds longer than control.

In some embodiments, an effective amount of an active agent (an agent that reduces the level and/or enzymatic activity of a lipid synthesis acyltransferase) is an amount that is effective to increase liver function by at least about 10%, at least about 20%, at least about 30%, at least about 40%, at least about 50%, at least about 60%, at least about 70%, at least about 80%, or more. In some embodiments, an effective amount of an active agent (an agent that reduces the level and/or enzymatic activity of a lipid synthesis acyltransferase) is an amount that is effective to reduce an elevated level of a serum marker of liver function by at least about 10%, at least about 20%, at least about 30%, at least about 40%, at least about 50%, at least about 60%, at least about 70%, at least about 80%, or more, or to reduce the level of the serum marker of liver function to within a normal range.

### DGAT1

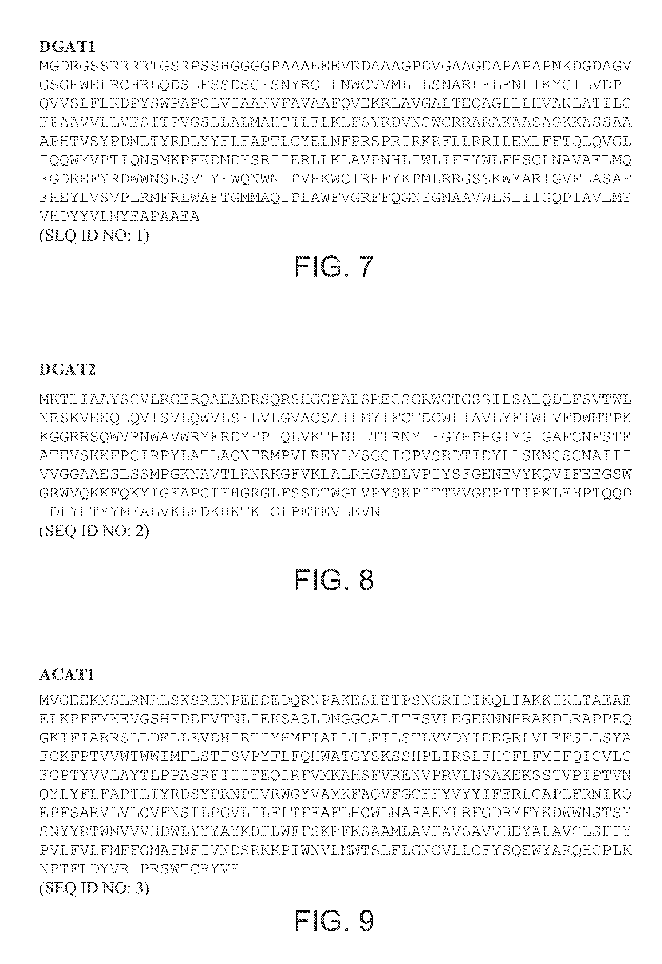

"DGAT1" refers to an enzyme that catalyzes the final reaction in triglyceride synthesis, e.g., DGAT1 catalyzes the transfer of coenzymeA-activated fatty acids to the 3 position of 1,2-diacylglycerols. As such, DGAT1 catalyzes the formation of triglycerides from diacylglycerol and acyl-CoA. See, e.g., U.S. Pat. No. 6,100,077 and Cases, et al. (1998) Proc. Nat. Acad. Sci. USA 95:13018-13023; and GenBank Accession Nos.. NP_036211 and AAH06263. "DGAT1" encompasses an enzymatically active polypeptide comprising an amino acid sequence having at least about 75%, at least about 80%, at least about 85%, at least about 90%, at least about 95%, at least about 98%, at least about 99%, or 100%, amino acid sequence identity to the amino acid sequence depicted in Figure 7 (SEQ ID NO:1).

### DGAT2

"DGAT2" refers to an enzyme that catalyzes the final reaction in triglyceride synthesis, e.g., DGAT2 catalyzes the transfer of coenzymeA activated fatty acids to the 3 position of 1,2-diacylglycerols. As such, DGAT2 catalyzes the formation of triglycerides from diacylglycerol and acyl-CoA. Amino acid sequences of DGAT2 polypeptides are known. See, e.g., U.S. Pat No. 6,822,141; Cases et al. (2001) J. Biol Chem., 276(42):38870-38876; U.S. Patent Publication No. 2006/0183210; and GenBank Accession No. NP 115953. "DGAT2" encompasses an enzymatically active polypeptide comprising an amino acid sequence having at least about 75%, at least about 80%, at least about 85%, at least about 90%, at least about 95%, at least about 98%, at least about 99%, or 100%, amino acid sequence identity to the amino acid sequence depicted in Figure 8 (SEQ ID NO:2).

### ACAT1

"ACAT1" (also referred to in the literatures as "SOAT1") refers an enzyme that catalyzes the covalent joining of cholesterol or oxysterols with long chain fatty acyl-coA moieties to form sterol esters. As such, ACAT1 catalyzes the formation of sterol esters using cholesterol or oxysterols as the acyl acceptor. Amino acid sequences of ACAT1 polypeptides are known in the art. See, e.g., U.S. Pat. No. 6,100,077; Buhman, et al. (2001) J. Biol. Chem. 276:40369-40372; and GenBank Accession No. NP 003092. The term "ACAT1" encompasses an enzymatically active polypeptide comprising an amino acid sequence having at least about 75%, at least about 80%, at least about 85%, at least about 90%, at least about 95%, at least about 98%, at least about 99%, or 100%, amino acid sequence identity to the amino acid sequence depicted in Figure 9 (SEQ ID NO:3).

### ACAT2

"ACAT2" (also referred to in the literature as "SOAT2") refers to an enzyme that catalyzes the covalent joining of cholesterol or oxysterols with long chain fatty acyl-coA moieties to form sterol esters. As such, ACAT2 catalyzes the formation of sterol esters using cholesterol or oxysterols as the acyl acceptor. Amino acid sequences of ACAT2 are known in the art. See, e.g., U.S. Pat. No. 6,869,937; Buhman, et al. (2001) J. Biol. Chem. 276:40369-40372; GenBank Accession No. NP_003569 and the genetic sequence as NM 003578. The term "ACAT2" encompasses an enzymatically active polypeptide comprising an amino acid sequence having at least about 75%, at least about 80%, at least about 85%, at least about 90%, at least about 95%, at least about 98%, at least about 99%, or 100%, amino acid sequence identity to the amino acid sequence depicted in Figure 10 (SEQ ID NO:4).

### Small molecule inhibitors

In some embodiments, an active agent that reduces the enzymatic activity of a lipid synthesis acyltransferase is a small molecule inhibitor, e.g., an agent that has a molecular weight of less than about 10 kD, less than about 5 kD, less than about 2.5 kD, less than about 2 kD, less than about 1 kD, less than about 0.5 kD, less than about 0.1 kD, or less than about 0.05 kD. Suitable small molecule active agents include organic compounds. Suitable small molecule active agents include agents that inhibit DGAT1 enzymatic activity, agents that inhibit DGAT2 enzymatic activity, agents that inhibit ACAT1 enzymatic activity, and agents that inhibit ACAT2 enzymatic activity.

### DGAT1 inhibitors

DGAT1 inhibitors suitable for use in treating an HCV infection include agents that are selective DGAT1 inhibitors, e.g., a suitable agent includes a compound that inhibits DGAT1 activity, but does not substantially inhibit DGAT2 enzymatic activity, e.g., the compound inhibits DGAT2 activity, if at all, by less than about 10%, less than about 5%, less than about 2%, or less than about 1% when used at a concentration that reduces the enzymatic activity of a DGAT1 enzyme by at least about 10% or more.

In some embodiments, a suitable DGAT1 inhibitor reduces an enzymatic activity of a DGAT1 polypeptide by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, or at least about 90%, compared to the enzymatic activity of the DGAT1 polypeptide in the absence of the inhibitor.

In some embodiments, a suitable DGAT1 inhibitor inhibits DGAT1 activity with an IC₅₀ of from about 1 nM to about 1 mM, e.g., from about 1 nM to about 10 nM, from about 10 nM to about 15 nM, from about 15 nM to about 25 nM, from about 25 nM to about 50 nM, from about 50 nM to about 75 nM, from about 75 nM to about 100 nM, from about 100 nM to about 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 450 nM, from about 450 nM to about 500 nM, from about 500 nM to about 750 nM, from about 750 nM to about 1 µM, from about 1 µM to about 10 µM, from about 10 µM to about 25 µM, from about 25 µM to about 50 µM, from about 50 µM to about 75 µM, from about 75 µM to about 100 µM, from about 100 µM to about 250 µM, from about 250 µM to about 500 µM, or from about 500 µM to about 1 mM.

Suitable DGAT1 inhibitors include those disclosed in, e.g., U.S. Patent Publication Nos. 2008/0096874, 2008/0090876, 2008/0182861, and 2008/0064717, and in U.S. Patent Nos. 7,423,156 and 7,317,125.

In some embodiments, a suitable DGAT1 inhibitor is an oxadiazole compound of the formula: 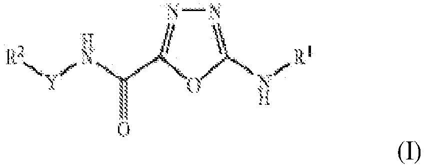 in which R¹ is an optionally substituted aryl or optionally substituted hetero aryl group; Y is a direct bond, or a group (CR⁴⁰R⁴¹)ₛ or -X6(CR⁴⁰R⁴¹)ₜ - where each R⁴⁰ and R⁴¹ is independently selected from hydrogen, (1-4C)alkyl, hydroxyl, halo, halo(1-4C)alkyl, amino, cyano, (1-4C)alkoxy, (1-4C)haloalkoxy or ((1-3)alkyl)CONH-, s is an integer of from 1 to 6 and t is an integer of from 1 to 6. R² is an optionally substituted aryl, an optionally substituted cycloalkyl or an optionally substituted heterocyclic group. Details on compound (I) are further described in US2008/0096874, incorporated herein by reference.

In some embodiments, a suitable DGAT1 inhibitor is a compound of the following formula: 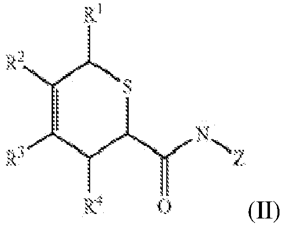 in which Z is selected from the group consisting of aryl and heteroaryl, in which each aryl and heteroaryl may be optionally substituted with 1 to 3 R⁵; R¹, R², R³, and R⁴ are independently selected from the group consisting of alkyl and alkoxy, in which R³ and R⁴ may be taken together to from an aryl ring that is optionally substituted with 1 to 3 R⁶. R⁵ is selected from the group consisting of alkyl, thioalkyl and halo; and R⁶ is selected from the group consisting of alkyl and alkoxy. Details on compound (II) are further described in US2008/0090876, incorporated herein by reference.

In some embodiments, a suitable DGAT1 inhibitor is a compound of the following formula III: 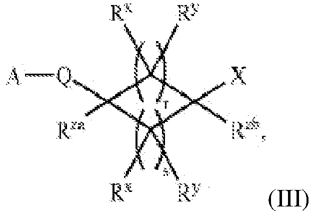 in which Q is a phenyl or a monocyclic heteroaryl; A is phenyl, or a 4-, 5-, 6- or 7-memebered monocyclic ring selected from the group consisting of heteroaryl and heterocycle; r and s are independently 1 or 2; X is X¹, -(CR^{k}R^{m})ᵤ-X¹, -(CR^{k}R^{m})ᵤ-C(O) -X¹, or -C(O) -X¹, in which X¹ is heterocycle or heteroaryl; q, t, u, v, and w, at each occurrence, are each independently 1, 2, 3, 4, 5, or 6; and R^{x}, R^{y}, R^{za}, R^{zb}, R^{k} and R^{m} at each occurrence, are independently hydrogen, alkyl, or haloalkyl. Further details of compound (III) can be found in US2008/0182861, incorporated herein by reference.

In other embodiments, a suitable DGAT1 inhibitor is a compound of the following formula (IV): 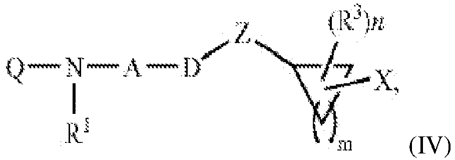
in which Q is -C(=Y)N(R²)(R^{2a}), -C(=W)(R^{b}), -R^{b}, -S(O)₂(R^{b}), or -C(O)O(R^{b}); R¹ and R^{2a} are each independently hydrogen or lower alkyl; R² is alkyl, aryl, heteroaryl, cycloalkyl, cycloalkyenyl, or heterocycle; R³ represents a substituent group selected from the group consisting of alkyl, haloalkyl, and halogen, m is 1, 2, 3, 4, or 5; n is 0, 1, or 2;
A and D are each a monocyclic ring selected from the group consisting of phenyl, heteroaryl, cycloalkyl, and cycloalkenyl; Z is C(O), C(H)(OH), C(alkyl)(OH), O, N(R^{b}), S(O), S(O)₂, or CH₂; X represents a substituent group selected from the group consisting of -C(O)OR⁵, -C(O)N(R⁵)₂, -CN, -C(=NOR⁵)N(R⁵)₂, -C(R⁶R⁷)OH, -C(O) -N(R⁵)(OR⁵), and tetrozolyl. R⁴, at each occurrence, is independently aryl, heteroaryl, cycloalkyl, cycloaklenyl, or heterocycle. R⁵, at each occurrence, is independently hydrogen, alkyl, or haloalkyl; R⁶ and R⁷ are independently hydrogen or alkyl, or R⁶ and R⁷ together with the carbon atom to which they are attached, form a three to six-membered, monocyclic ring selected from the group consisting of cycloalkyl and cycloalkenyl. R^{b}, at each occurrence, is independently alkyl, ahloalkyl, or R⁴. Further details on compound (IV) can be found in US2008/0064717, disclosure of which is incorporated herein by reference.
In other embodiments, a suitable DGAT1 inhibitor is a compound of the following formula (V): 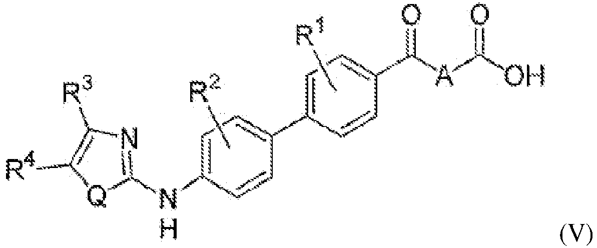 in which, Q is O, S, or NR⁵; A is a linker selected from 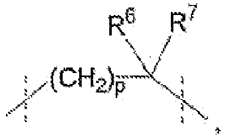 in which p is 1 or 2 and 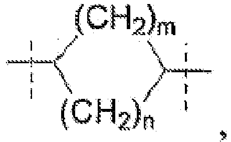
in which m is 0, and n is 1, 2, 3, or 4, or m is 1 and n is 1,2, or 3, and in which the linker is optionally substituted by one or more R⁸ groups;
R¹ and R² are independently selected from hydrogen, halo, (C₁-C₆)alkyl, and (C₁-C₆)alkoxy; R³ is selected from hydrogen, (C₁-C₆)alkyl optionally substituted by hydroxy, and phenyl optionally substituted with (C₁-C₆)alkyl, (C₁-C₆)alkoxy, or halo. R⁴ is selected from hydrogen, nitro, and (C₁-C₆)alkyl. R³ and R⁴, when taken together with the carbon atoms to which they are attached, may form a benzene ring with optional substitutions. R⁵ is hydrogen or (C₁-C₆)alkyl; R⁶ is hydrogen; R⁷ is hydrogen or (C₁-C₆)alkyl optionally substituted with (C₁-C₆)alkoxy, bis[(C₁-C₆)alkyl]amino or phenyl optionally substituted with halo, (C₁-C₆)alkyl, or (C₁-C₆)alkoxy, or cyano;
R⁶ and R⁷ may also be both (C₁-C₆)alkyl or together with the carbon atom to which they are attached, form a 3- to 5-membered carbocyclic ring, or a 6-membered ring represented by 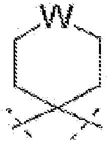 in which W is CH₂, C(CH₃)₂, O, NR⁹, X, or SO₂. R⁹ is hydrogen or (C₁-C₆)alkyl.

A further exemplary DGAT1 inhibitor is a compound of the formula: 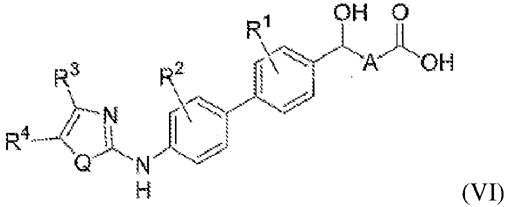 or 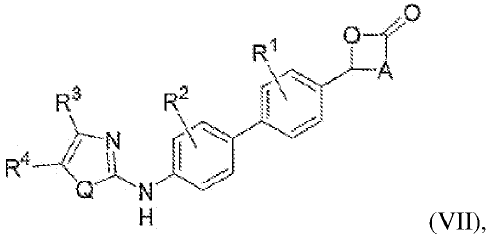 in which Q, A, and R¹-R⁴ have the meanings as described above for formula (V). Details of compounds of formula (V), (VI), and (VII) can be found in WO2004/100881, disclosure of which is incorporated herein by reference.

### DGAT2 inhibitors

DGAT2 inhibitors suitable for use in treating an HCV infection include agents that are selective DGAT2 inhibitors, e.g., a suitable agent includes a compound that inhibits DGAT2 activity, but does not substantially inhibit DGAT1 enzymatic activity, e.g., the compound inhibits DGAT1 activity, if at all, by less than about 10%, less than about 5%, less than about 2%, or less than about 1% when used at a concentration that reduces the enzymatic activity of a DGAT2 enzyme by at least about 10% or more.

In some embodiments, a suitable DGAT2 inhibitor reduces an enzymatic activity of a DGAT2 polypeptide by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, or at least about 90%, compared to the enzymatic activity of the DGAT2 polypeptide in the absence of the inhibitor.

In some embodiments, a suitable DGAT2 inhibitor inhibits DGAT2 activity with an IC₅₀ of from about 1 nM to about 1 mM, e.g., from about 1 nM to about 10 nM, from about 10 nM to about 15 nM, from about 15 nM to about 25 nM, from about 25 nM to about 50 nM, from about 50 nM to about 75 nM, from about 75 nM to about 100 nM, from about 100 nM to about 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 450 nM, from about 450 nM to about 500 nM, from about 500 nM to about 750 nM, from about 750 nM to about 1 µM, from about 1 µM to about 10 µM, from about 10 µM to about 25 µM, from about 25 µM to about 50 µM, from about 50 µM to about 75 µM, from about 75 µM to about 100 µM, from about 100 µM to about 250 µM, from about 250 µM to about 500 µM, or from about 500 µM to about 1 mM.

Suitable DGAT2 inhibitors include those disclosed in US Pat Pub No. 2008/0166420, WO2006/132879, and Gangi et al. (2004) J. Lipid Res. 45:1835-1845.

In some embodiments, a suitable DGAT2 inhibitor is also a DGAT1 inhibitor.

A suitable DGAT2 inhibitor is a polymethoxylated flavone (PMF). PMF include polymethoxylated, mono- methoxylated flavones and/or hydroxylated flavones. In one embodiment, the PMF is tangeretin. In another embodiment the PMF is nobiletin. PMF include citrus flavonoids. Other suitable PMF include limocitrin, limocitrin derivatives, quercetin and quercetin derivatives, including, but not limited to, limocitrin-3,7,4'-trimethylether (5-hydroxy-3,7,8,3',4'-pentamethoxyfiavone); limocitrin-3,5,7,4'-tetramethylether (3,5,7,8,3',4'-hexamethoxyflavone); limocitrin-3,5,7,4'-tetraethylether (8,3¹- dimethoxy-3,5,7,4'-hexamethoxyflavone); limocitrin-3,7,4'-trimethylether-5- acetate; quercetin tetramethylether (5-hydroxy-3,7,3',4'-tetramethoxyflavone); quercetin-3,5-dimethylether-7,3',4'-tribenzyl ether; quercetin pentamethyl ether (3, 5,7,3 ',4'-pentamethoxyflavone); quercetin-5,7,3',4'-tetramethylether-3- acetate; and quercetin-5,7,3',4'-tetramethylether (3-hydroxy-5,7,3',4'-tetramethoxyflavone); and the naturally occurring polymethoxyflavones: 3,5,6,7,8,3',4'-heptan-ethoxyflavone; 5-desmethylnobiletin (5-hydroxy- 6,7,8,3',4'-pentamethoxyflavone); tetra-0-methylisoscutellarein (5,7,8,4'- tetramethoxyflavone); 5-desmethylsinensetin (5-hydroxy-6,7,3',4'- tetramethoxyflavone); and sinensetin (5,6,7,3',4'-pentamethoxyflavone). Another suitable PMF is tocotrienol. Further details on compositions that inhibit DGAT2 can be found in US Pat Pub No. 2008/0166420, the disclosure of which is incorporated herein by reference.

Some exemplary PMF that canbe used to inhibit DGAT2 are of the following structural formulae: 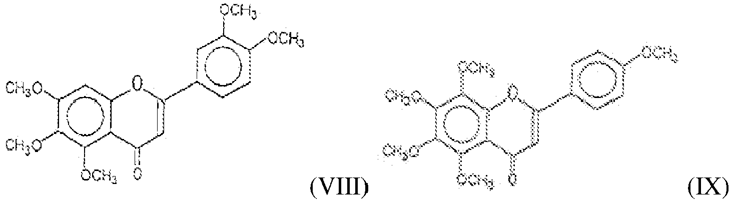 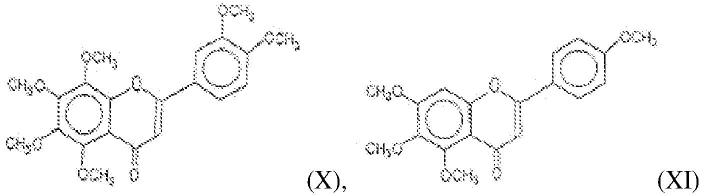 in which compound VIII is sinesetin, compound IX is tangeretin, compound X is nobiletin, and compound XI is tetramethyl-O-scutellarein. Further details on PMF molecules can be found in Green et al. (2007) Biomed. Chromatography 21:48-54.

Suitable DGAT2 inhibitors include niacin, also known as vitamin B₃, which is a watersoluble vitamin with the molecular formula C₆H₅NO₂. It is a derivative of pyridine, with a carboxyl group at the 3-position. Other forms of vitamin B₃ include the corresponding amide, nicotinamide ("niacinamide"), as well as more complex amides and a variety of esters. The terms niacin, nicotinamide, and vitamin B₃ are often used interchangeably to refer to any one of this family of molecules.

### ACAT inhibitors

ACAT1 inhibitors suitable for use in treating an HCV infection include agents that are selective ACAT1 inhibitors, e.g., a suitable agent includes a compound that inhibits ACAT1 activity, but does not substantially inhibit ACAT2 enzymatic activity, e.g., the compound inhibits ACAT2 activity, if at all, by less than about 10%, less than about 5%, less than about 2%, or less than about 1% when used at a concentration that reduces the enzymatic activity of an ACAT1 enzyme by at least about 10% or more.

ACAT inhibitors suitable for use in treating an HCV infection include agents that inhibit both ACAT1 and ACAT2.

In some embodiments, a suitable ACAT1 inhibitor reduces an enzymatic activity of an ACAT1 polypeptide by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, or at least about 90%, compared to the enzymatic activity of the ACAT1 polypeptide in the absence of the inhibitor.

In some embodiments, a suitable ACAT1 inhibitor inhibits ACAT1 activity with an IC₅₀ of from about 1 nM to about 1 mM, e.g., from about 1 nM to about 10 nM, from about 10 nM to about 15 nM, from about 15 nM to about 25 nM, from about 25 nM to about 50 nM, from about 50 nM to about 75 nM, from about 75 nM to about 100 nM, from about 100 nM to about 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 450 nM, from about 450 nM to about 500 nM, from about 500 nM to about 750 nM, from about 750 nM to about 1 µM, from about 1 µM to about 10 µM, from about 10 µM to about 25 µM, from about 25 µM to about 50 µM, from about 50 µM to about 75 µM, from about 75 µM to about 100 µM, from about 100 µM to about 250 µM, from about 250 µM to about 500 µM, or from about 500 µM to about 1 mM.

ACAT2 inhibitors suitable for use in treating an HCV infection include agents that are selective ACAT2 inhibitors, e.g., a suitable agent includes a compound that inhibits ACAT2 activity, but does not substantially inhibit ACAT1 enzymatic activity, e.g., the compound inhibits ACAT1 activity, if at all, by less than about 10%, less than about 5%, less than about 2%, or less than about 1% when used at a concentration that reduces the enzymatic activity of an ACAT2 enzyme by at least about 10% or more.

In some embodiments, a suitable ACAT2 inhibitor reduces an enzymatic activity of an ACAT2 polypeptide by at least about 5%, at least about 10%, at least about 15%, at least about 20%, at least about 25%, at least about 30%, at least about 35%, at least about 40%, at least about 45%, at least about 50%, at least about 60%, at least about 70%, at least about 80%, or at least about 90%, compared to the enzymatic activity of the ACAT2 polypeptide in the absence of the inhibitor.

In some embodiments, a suitable ACAT2 inhibitor inhibits ACAT2 activity with an IC₅₀ of from about 1 nM to about 1 mM, e.g., from about 1 nM to about 10 nM, from about 10 nM to about 15 nM, from about 15 nM to about 25 nM, from about 25 nM to about 50 nM, from about 50 nM to about 75 nM, from about 75 nM to about 100 nM, from about 100 nM to about 150 nM, from about 150 nM to about 200 nM, from about 200 nM to about 250 nM, from about 250 nM to about 300 nM, from about 300 nM to about 350 nM, from about 350 nM to about 400 nM, from about 400 nM to about 450 nM, from about 450 nM to about 500 nM, from about 500 nM to about 750 nM, from about 750 nM to about 1 µM, from about 1 µM to about 10 µM, from about 10 µM to about 25 µM, from about 25 µM to about 50 µM, from about 50 µM to about 75 µM, from about 75 µM to about 100 µM, from about 100 µM to about 250 µM, from about 250 µM to about 500 µM, or from about 500 µM to about 1 mM.

Exemplary ACAT inhibitors include those disclosed in US Patent Pub No. 2007/0155832, USPN 5,397,781, USPN 5,405,873, USPN 5,387,600, WO94/26702, and Krause et al., "ACAT Inhibitors: Physiologic Mechanisms for Hypolipidemic and Anti-A Theroschlerotic Activities in Experimental Animals" in Inflammation: Mediators and Pathways ACAT Inhibitors, Ruffalo et al., Eds. CRC Press, Boca Raton 1995 Chapter 6:173-197.

In certain cases, one or more DGAT1 or 2 inhibitors described above can also be used to inhibit ACAT1 and/or ACAT2 in the subject method. In some embodiments, a suitable ACAT1 inhibitor is also an ACAT2 inhibitor.

Any ACAT inhibitor known in the art that inhibits the intracellular esterification of dietary cholesterol by the enzyme acyl CoA: cholesterol acyltransferase can be used. Such inhibition is determined readily according to standard assays, such as the method described in Heider et al. (1983) J. of Lipid Res. 24:1127.

Examples of suitable ACAT inhibitors include, but are not limited to, those described in U.S. Pat. No. 5,510,379 (carboxysulfonates), WO 96/26948 and WO 96/10559 (urea derivatives). Additional examples include Avasimibe (Pfizer), CS-505 (Sankyo), KY-505 (Sanyo), SMP797 (Sumitomo), Eflucimibe (Eli Lilly and Pierre Fabre), HL-004, lecimibide (DuP-128) and CL-277082 (N-(2,4-difluorophenyl)-N-[[4-(2,2-dimethylpropyl)phenyl]methyl]-N-heptyl- urea), melinamide (French Pat No. 1,476,569), serum amyloid isoform 2.1/1.1 (US Pat Pub No. 2008/0221028), TS-962 (Taisho Pharmaceutical Co. Ltd), as well as F-1394, CS-505, F-12511, HL-004, K-10085 and YIC-C8-434.

Other ACAT inhibitors include those disclosed in: Drugs of the Future (1999) 24:9-15; Nicolosi et al. (1998) Atherosclerosis 137:77-85; Ghiselli et al. (1998) Cardiovasc. Drug Rev., 16:16-30; Smith, C. et al. (1996) Bioorg. Med. Chem. Lett, 6: 47-50; Krause et al. (1995) Editor(s): Ruffolo, Robert R., Jr.; Hollinger, Mannfred A., Inflammation: Mediators Pathways, 173-98, Publisher: CRC, Boca Raton, Fla.; Sliskovic et al. (1994) Curr. Med. Chem. 1:204-25; and Stout et al. (1995) Chemtracts: Org. Chem. 8:359-62.

In other embodiments, inhibitors of ACAT-catalyzed cholesterol esterification also include the local anesthetics lidocaine, tetracaine, benzocaine and dibucaine, the tranquilizer chlorpromazine, the hypolipidemics clofibrate and benzafibrate, progesteron, ethyl ester of (z)-N-(1-oxo-9- octadecenyl)-D,L-tryptophan, (3-decyl-dimethyl silyl)-N-[Z-(4-methylphenyl)-1-phenethyl] propionamide), and N,-2,4-difluorophenyl-N-n-heptyl-N-(4-neopentyl) benzyl urea.

Other inhibitors of ACAT include: 2,2-dimethyl-N-(2,4,6-trimethoxyphenyl)dodecanamide disclosed in U.S. Pat. No. 4,716,175; and N-[2,6-bis(1-methylethyl)phenyl]--N'-[[1-(4-dimethylaminophenyl)cyclopentyl]methyl]urea disclosed in U.S. Pat. No. 5,015,644; 2,6-bis(1-methyl-ethyl)phenyl[[2,4,6-tris(1-methylethyl)phenyl]-acetyl] sulfamate.

For more examples of known ACAT inhibitor, see P. Chang et al. (2000) "Current, New and Future Treatments in Dyslipidaemia and Atherosclerosis", Drugs 60(1); 55-93. Generally, a total daily dosage of ACAT inhibitor(s) can range from about 0.1 to about 1000 mg/day in single or 2-4 divided doses.

An exemplary inhibitor that can be used to inhibit ACAT1/2 is of the following structural formula (Formula XII):

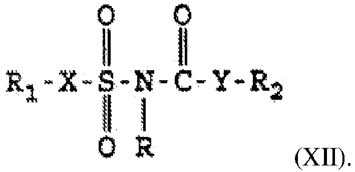

X and Y of Formula XII are selected from oxygen, sulfur, and (CR'R")ₙ, in which n is an integer from 1 to 4 and R' and R" are each independently hydrogen, alkyl, alkoxy, halogen, hydroxyl, acyloxy, cycloalkyl, phenyl optionally substituted. R₁ and R₂ are each independently selected from phenyl or phenoxy, 1- or 2-naphthyl, arylaklyl, alkyl chain, adamantyl, or a cycloalkyl. More details on compound XII can be found in WO94/26702 and US Pat Pub No. 2007/0155832, the disclosures of which are incorporated herein by reference.

In another embodiment, an exemplary inhibitor that can be used to inhibit ACAT1/2 is of the following structural formula: 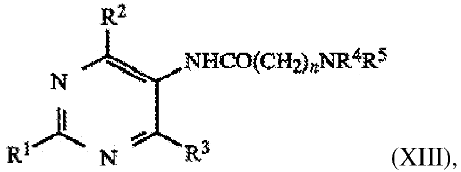
in which n represents an integer from 1 to 6;
R¹ represents a hydrogen atom, an alkyl group of straight or branched chain having 1 to 4 carbon atoms, NR⁶R⁷, SR⁸, or OR⁸; R² represents a hydrogen atom, NR⁹R¹⁰, SR¹¹, OR¹¹, an alkyl group of straight of branched chain having 1 to 6 carbon atoms, or halogen atom; R³ represents a hydrogen atom, NR¹²R¹³, SR¹⁴, OR¹⁴, an alkyl group of straight of branched chain having 1 to 6 carbon atoms, or halogen atom; R⁴ and R⁵ are identical or different and each represents a group selected from the group consisting of hydrogen atom, an alkyl group of straight or branched chain having 1 to 12 carbon atoms, a benzyl group, a cycloalkyl group having 3 to 10 carbon atoms, and a phenyl group; R⁴ and R⁵ may also with the nitrogen atom to which they are bonded, form a piperazine ring substituted with a phenyl group, or a tetrahydroquinoline ring; R⁶, R⁷, and R⁸ each represents a hydrogen atom, or an alkyl group of straight or branched chain having 1 to 4 carbon atoms; R⁹, R¹⁰, R¹¹, R¹², R¹³, and R¹⁴ each represents a hydrogen atom, a phenyl group, a benzyl group, or an alkyl group of straight or branched chain having 1 to 10 carbon atoms; R⁹ and R¹⁰ or R¹² and R¹³, together with the nitrogen atom to which they are bonded, may form a morpholine ring or a piperazine ring. More details on compound XIII can be found in USPN 5,397,781, the disclosure of which is incorporated herein by reference.

In certain embodiments, an inhibitor of ACAT1/2 is of the following structural formula: 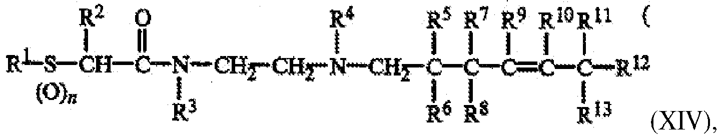
in which n represents 0, 1, or 2;
R¹ represents an aryl group or an aromatic heterocyclic group which amy optionally be substituted; R² represents hydrogen atom or a lower alkyl group; R³ represents hydrogen atom or alower alkyl group; R⁴ represents an alkyl group, an alkenyl group, or an alkanoyl group, having 3 to 10 carbon atoms; R⁵, R⁶, R⁷, and R⁸ each represents hydrogen atom or a lower alkyl group; R⁵ and R⁷ or R⁶ and R⁸ may be combined together to form a single bond; R⁹ and R¹⁰ each represents a hydrogen atom or a lower alkyl group, or both are combined together to form a single bond; R¹¹ and R¹² each represents hydrogen atom or a lower alkyl group, or both are combined together to form a cycloalkane together with the carbon atom adjacent thereto; R¹³ represents a hydrogen atom, a lower alkyl group, or a lower alkoxy group. More details on compound XIV can be found in USPN 5,405,873, the disclosure of which is incorporated herein by reference.

In other embodiments, an inhibitor of ACAT1/2 is one of the following structural formulae: 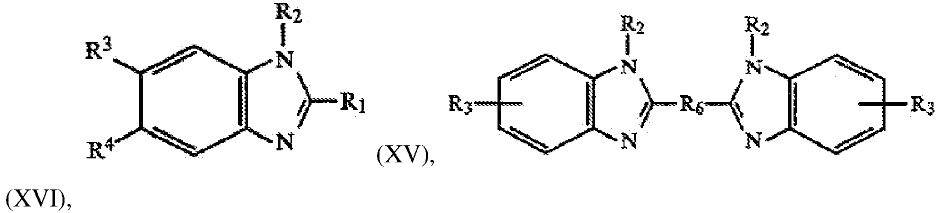 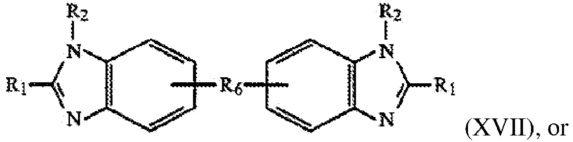 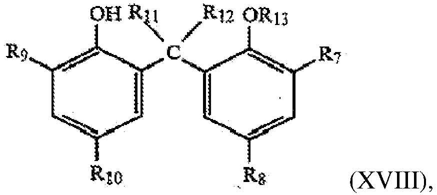 where R₁ represents a hydrogen atom, an alkyl, an aryl, a mercapto, an alkylthio, an alkenylthio, an arylthio or a heterocyclo group; R₂ represents a hydrogen atom, or an alkyl group, provided that the alkyl group is not substituted by a hydroxyl group; R₃ and R₄ each represents a hydrogen atom, a halogen atom, a nitro group, R₅O-, R₅CONH-, R₅NHCO-, (R₅)₂NCO-, R₅SO₂NH-, R₅NHSO₂-, R₅OCO-, R₅COO-, or R₅NHCONH-, in which R₅ represents an alkyl or an aryl group; R⁶ represents a divalent group. R₇, R₈, R₉, and R₁₀ each represents a alkyl a cycloalkyl group, -(C(CH₃)₂)ₖ-CH₂-mCOOR₁₄ or -(C(CH₃)₂)ₖ-(CH₂)mCON(R₁₄)₂ where k represents 0 or 1, m represents an integer of 0 to 4 and R₁₄ represents a lower alkyl group; R₁₁ and R₁₂ each represents a hydrogen atom, an alkyl, an aryl, or an aralkyl group; R₁₃ represents a hydrogen atom, a lower alkyl, an aralkyl, an acyl, an alkyl- or arylsulfonyl group, or -(CH₂)ₙCOOR₁₅ where n represents an integer of 0 to 2 and R₁₅ represents a lower alkyl group. More details on compounds XV to XVIII can be found in USPN 5,387,600, the disclosure of which is incorporated herein by reference.

### Interfering Nucleic Acids

In some embodiments, an active agent that reduces the level of a lipid synthesis acyltransferase, and thus is suitable for use in a subject method, is an interfering RNA that specifically reduces the level of a lipid synthesis acyltransferase. In one embodiment, reduction of an acyltransferase protein gene product level is accomplished through RNA interference (RNAi) by contacting a cell with a small nucleic acid molecule, such as a short interfering nucleic acid (siNA), a short interfering RNA (siRNA), a double-stranded RNA (dsRNA), a micro-RNA (miRNA), or a short hairpin RNA (shRNA) molecule, or modulation of expression of a small interfering RNA (siRNA) so as to provide for decreased levels of an acyltransferase protein gene product. siRNAs that inhibits the production of DGAT2 are found in US Pat Pub No. 2008/0113369.

The term "short interfering nucleic acid," "siNA," "short interfering RNA," "siRNA," "short interfering nucleic acid molecule," "short interfering oligonucleotide molecule," or "chemically-modified short interfering nucleic acid molecule" as used herein refers to any nucleic acid molecule capable of inhibiting or down regulating gene expression, for example by mediating RNA interference "RNAi" or gene silencing in a sequence-specific manner. Design of RNAi molecules when given a target gene is routine in the art. See also US 2005/0282188 (which is incorporated herein by reference) as well as references cited therein. See, e.g., Pushparaj et al. Clin Exp Pharmacol Physiol. 2006 May-Jun;33(5-6):504-10; Lutzelberger et al. Handb Exp Pharmacol. 2006;(173):243-59; Aronin et al. Gene Ther. 2006 Mar;13(6):509-16; Xie et al. Drug Discov Today. 2006 Jan;11(1-2):67-73; Grunweller et al. Curr Med Chem. 2005;12(26):3143-61; and Pekaraik et al. Brain Res Bull. 2005 Dec 15;68(1-2):115-20. Epub 2005 Sep 9.

Methods for design and production of siRNAs to a desired target are known in the art, and their application to acyltransferase genes for the purposes disclosed herein will be readily apparent to the ordinarily skilled artisan, as are methods of production of siRNAs having modifications (e.g., chemical modifications) to provide for, e.g., enhanced stability, bioavailability, and other properties to enhance use as therapeutics. In addition, methods for formulation and delivery of siRNAs to a subject are also well known in the art. See, e.g., US 2005/0282188; US 2005/0239731; US 2005/0234232; US 2005/0176018; US 2005/0059817; US 2005/0020525; US 2004/0192626; US 2003/0073640; US 2002/0150936; US 2002/0142980; and US2002/0120129, each of which are incorporated herein by reference.

Publicly available tools to facilitate design of siRNAs are available in the art. See, e.g., DEQOR: Design and Quality Control of RNAi (available on the internet at cluster-1.mpi-cbg.de/Deqor/deqor.html). See also, Henschel et al. Nucleic Acids Res. 2004 Jul 1;32(Web Server issue):W113-20. DEQOR is a web-based program which uses a scoring system based on state-of-the-art parameters for siRNA design to evaluate the inhibitory potency of siRNAs. DEQOR, therefore, can help to predict (i) regions in a gene that show high silencing capacity based on the base pair composition and (ii) siRNAs with high silencing potential for chemical synthesis. In addition, each siRNA arising from the input query is evaluated for possible cross-silencing activities by performing BLAST searches against the transcriptome or genome of a selected organism. DEQOR can therefore predict the probability that an mRNA fragment will cross-react with other genes in the cell and helps researchers to design experiments to test the specificity of siRNAs or chemically designed siRNAs.

siNA (e.g., siRNA) molecules can be of any of a variety of forms. For example the siNA can be a double-stranded polynucleotide molecule comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. siNA can also be assembled from two separate oligonucleotides, where one strand is the sense strand and the other is the antisense strand, wherein the antisense and sense strands are self-complementary. In this embodiment, each strand generally comprises nucleotide sequence that is complementary to nucleotide sequence in the other strand; such as where the antisense strand and sense strand form a duplex or double stranded structure, for example wherein the double stranded region is about 15 base pairs to about 30 base pairs, e.g., about 15, 16, 17, 18, 19, 20, 21, 22, 23, 24, 25, 26, 27, 28, 29 or 30 base pairs; the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof (e.g., about 15 nucleotides to about 25 or more nucleotides of the siNA molecule are complementary to the target nucleic acid or a portion thereof).

Alternatively, the siNA (e.g., siRNA) can be assembled from a single oligonucleotide, where the self-complementary sense and antisense regions of the siNA are linked by a nucleic acid-based or non-nucleic acid-based linker(s). The siNA can be a polynucleotide with a duplex, asymmetric duplex, hairpin or asymmetric hairpin secondary structure, having self-complementary sense and antisense regions, wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof.

The siNA can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siNA molecule capable of mediating RNAi. The siNA can also comprise a single stranded polynucleotide having nucleotide sequence complementary to nucleotide sequence in a target nucleic acid molecule or a portion thereof (e.g., where such siNA molecule does not require the presence within the siNA molecule of nucleotide sequence corresponding to the target nucleic acid sequence or a portion thereof), wherein the single stranded polynucleotide can further comprise a terminal phosphate group, such as a 5'-phosphate (see for example Martinez et al., 2002, Cell., 110, 563-574 and Schwarz et al., 2002, Molecular Cell, 10, 537-568), or 5',3'-diphosphate.

In certain embodiments, the siNA molecule contains separate sense and antisense sequences or regions, wherein the sense and antisense regions are covalently linked by nucleotide or non-nucleotide linkers molecules as is known in the art, or are alternately non-covalently linked by ionic interactions, hydrogen bonding, van der Waals interactions, hydrophobic interactions, and/or stacking interactions. In certain embodiments, the siNA molecules comprise nucleotide sequence that is complementary to nucleotide sequence of a target gene. In another embodiment, the siNA molecule interacts with nucleotide sequence of a target gene in a manner that causes inhibition of expression of the target gene.

As used herein, siNA molecules need not be limited to those molecules containing only RNA, but further encompasses chemically-modified nucleotides and non-nucleotides. In certain embodiments, the short interfering nucleic acid molecules of the invention lack 2'-hydroxy (2'-OH) containing nucleotides. siNAs do not necessarily require the presence of nucleotides having a 2'-hydroxy group for mediating RNAi and as such, siNA molecules of the invention optionally do not include any ribonucleotides (e.g., nucleotides having a 2'-OH group). Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions. The modified short interfering nucleic acid molecules of the invention can also be referred to as short interfering modified oligonucleotides "siMON."

As used herein, the term siNA is meant to be equivalent to other terms used to describe nucleic acid molecules that are capable of mediating sequence specific RNAi, for example short interfering RNA (siRNA), double-stranded RNA (dsRNA), micro-RNA (miRNA), short hairpin RNA (shRNA), short interfering oligonucleotide, short interfering nucleic acid, short interfering modified oligonucleotide, chemically-modified siRNA, post-transcriptional gene silencing RNA (ptgsRNA), and others. In addition, as used herein, the term RNAi is meant to be equivalent to other terms used to describe sequence specific RNA interference, such as post transcriptional gene silencing, translational inhibition, or epigenetics. For example, siNA molecules of the invention can be used to epigenetically silence a target gene at the post-transcriptional level and/or the pre-transcriptional level. In a non-limiting example, epigenetic regulation of gene expression by siNA molecules of the invention can result from siNA mediated modification of chromatin structure or methylation pattern to alter gene expression (see, for example, Verdel et al., 2004, Science, 303, 672-676; Pal-Bhadra et al., 2004, Science, 303, 669-672; Allshire, 2002, Science, 297, 1818-1819; Volpe et al., 2002, Science, 297, 1833-1837; Jenuwein, 2002, Science, 297, 2215-2218; and Hall et al., 2002, Science, 297, 2232-2237).

siNA molecules contemplated herein can comprise a duplex forming oligonucleotide (DFO) see, e.g., WO 05/019453; and US 2005/0233329, which are incorporated herein by reference). siNA molecules also contemplated herein include multifunctional siNA, (see, e.g., WO 05/019453 and US 2004/0249178). The multifunctional siNA can comprise sequence targeting, for example, two regions of Skp2.

siNA molecules contemplated herein can comprise an asymmetric hairpin or asymmetric duplex. By "asymmetric hairpin" as used herein is meant a linear siNA molecule comprising an antisense region, a loop portion that can comprise nucleotides or non-nucleotides, and a sense region that comprises fewer nucleotides than the antisense region to the extent that the sense region has enough complementary nucleotides to base pair with the antisense region and form a duplex with loop. For example, an asymmetric hairpin siNA molecule can comprise an antisense region having length sufficient to mediate RNAi in a cell or in vitro system (e.g. about 15 to about 30, or about 15, 16, 17, 18, 19, 20, 21, 22, 23, 24, 25, 26, 27, 28, 29, or 30 nucleotides) and a loop region comprising about 4 to about 12 (e.g., about 4, 5, 6, 7, 8, 9, 10, 11, or 12) nucleotides, and a sense region having about 3 to about 25 (e.g., about 3, 4, 5, 6, 7, 8, 9, 10, 11, 12, 13, 14, 15, 16, 17, 18, 19, 20, 21, 22, 23, 24, or 25) nucleotides that are complementary to the antisense region. The asymmetric hairpin siNA molecule can also comprise a 5'-terminal phosphate group that can be chemically modified. The loop portion of the asymmetric hairpin siNA molecule can comprise nucleotides, non-nucleotides, linker molecules, or conjugate molecules as described herein.

By "asymmetric duplex" as used herein is meant a siNA molecule having two separate strands comprising a sense region and an antisense region, wherein the sense region comprises fewer nucleotides than the antisense region to the extent that the sense region has enough complementary nucleotides to base pair with the antisense region and form a duplex. For example, an asymmetric duplex siNA molecule of the invention can comprise an antisense region having length sufficient to mediate RNAi in a cell or in vitro system (e.g. about 15 to about 30, or about 15, 16, 17, 18, 19, 20, 21, 22, 23, 24, 25, 26, 27, 28, 29, or 30 nucleotides) and a sense region having about 3 to about 25 (e.g., about 3, 4, 5, 6, 7, 8, 9, 10, 11, 12, 13, 14, 15, 16, 17, 18, 19, 20, 21, 22, 23, 24, or 25) nucleotides that are complementary to the antisense region.

Stability and/or half-life of siRNAs can be improved through chemically synthesizing nucleic acid molecules with modifications (base, sugar and/or phosphate) can prevent their degradation by serum ribonucleases, which can increase their potency (see e.g., Eckstein et al., International Publication No. WO 92/07065; Perrault et al., 1990 Nature 344, 565; Pieken et al., 1991, Science 253, 314; Usman and Cedergren, 1992, Trends in Biochem. Sci. 17, 334; Usman et al., International Publication No. WO 93/15187; and Rossi et al., International Publication No. WO 91/03162; Sproat, U.S. Pat. No. 5,334,711; Gold et al., U.S. Pat. No. 6,300,074; and Burgin et al., supra; all of which are incorporated by reference herein, describing various chemical modifications that can be made to the base, phosphate and/or sugar moieties of the nucleic acid molecules described herein. Modifications that enhance their efficacy in cells, and removal of bases from nucleic acid molecules to shorten oligonucleotide synthesis times and reduce chemical requirements are desired.

siNA molecules can be provided as conjugates and/or complexes, e.g., to facilitate delivery of siNA molecules into a cell. Exemplary conjugates and/or complexes include those composed of an siNA and a small molecule, lipid, cholesterol, phospholipid, nucleoside, antibody, toxin, negatively charged polymer (e.g., protein, peptide, hormone, carbohydrate, polyethylene glycol, or polyamine). In general, the transporters described are designed to be used either individually or as part of a multi-component system, with or without degradable linkers. These compounds can improve delivery and/or localization of nucleic acid molecules into cells in the presence or absence of serum (see, e.g., US 5,854,038). Conjugates of the molecules described herein can be attached to biologically active molecules via linkers that are biodegradable, such as biodegradable nucleic acid linker molecules.

### DGAT1 interfering RNA

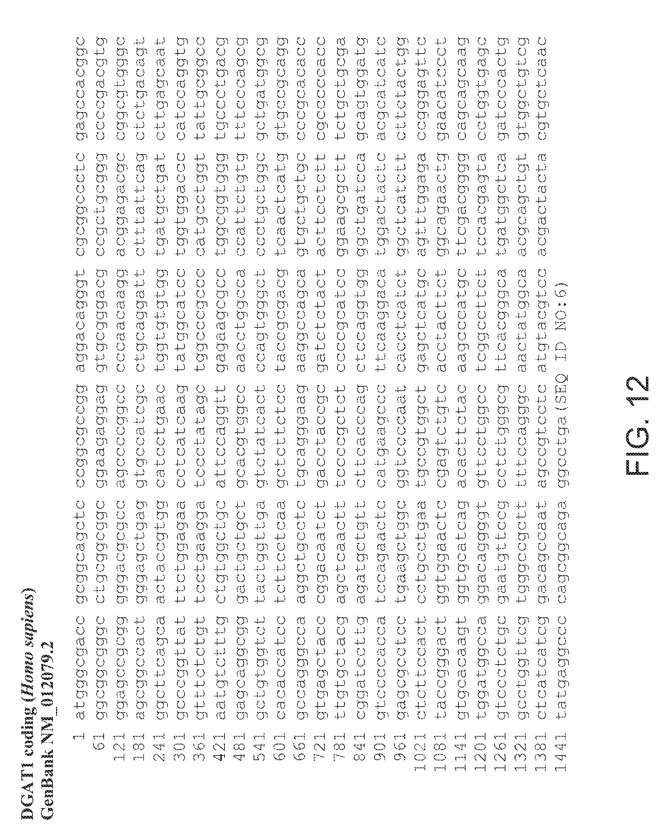

Interfering RNA that reduces the level of a DGAT1 polypeptide in a cell includes a nucleic acid 12 to 80 nucleobases in length targeted to at least an 8 nucleobase portion of the nucleotide sequence depicted in Figure 10, encoding diacylglycerol acyltransferase 1, wherein the nucleic acid comprises a nucleotide sequence that is at least 80%, at least 85%, at least 90%, at least 95%, at least 98%, at least 99 at least, or 100%, complementary to the nucleotide sequence depicted in Figure 12 (SEQ ID NO:6).

Interfering RNA that reduces the level of a DGAT1 polypeptide in a cell includes a nucleic acid 12 to 80 nucleobases in length targeted to at least an 8 nucleobase portion of the nucleotide sequence set forth in SEQ ID NO: 4 of U.S. Patent No. 7,414,033, encoding diacylglycerol acyltransferase 1, wherein the nucleic acid comprises a nucleotide sequence that is at least 80%, at least 85%, at least 90%, at least 95%, at least 98%, at least 99 at least, or 100%, complementary to the nucleotide sequence set forth in SEQ ID NO:4 of U.S. Patent No. 7,414,033.

Exemplary antisense RNA that reduces the level of a DGAT1 polypeptide in a cell include:
5'-GCCCAUGGCCUCAGCCCGCA-3' (SEQ ID NO:7);
5'-ACGCCGGCGUCUCCGUCCUU-3' (SEQ ID NO:8);
5'-CUGCAGGCGAUGGCACCUCA-3' (SEQ ID NO:9); and
5'-CUCCCAGCUGGCAUCAGACU-3' (SEQ ID NO:10).
Exemplary siRNA that reduces the level of a DGAT1 polypeptide in a cell include:
5'-CUUGAGCAAUGCCCGGUUA-3' (SEQ ID NO:11);
5'-CAAUAGCCGUCCUCAUGUA-3' (SEQ ID NO:12);
5'-UCAAGGACAUGGACUACUC-3' (SEQ ID NO:13); and
5'-GCUGUGGUCUUACUGGUUG -3' (SEQ ID NO:14).

### DGAT2

Interfering RNA that reduces the level of a DGAT1 polypeptide in a cell includes a nucleic acid 12 to 80 nucleobases in length targeted to at least an 8 nucleobase portion of the nucleotide sequence set forth in SEQ ID NO: 4 of U.S. Patent Publication no. 2005/0272680, encoding diacylglycerol acyltransferase-2, wherein the nucleic acid comprises a nucleotide sequence that is at least 80%, at least 85%, at least 90%, at least 95%, at least 98%, at least 99 at least, or 100%, complementary to the nucleotide sequence set forth in SEQ ID NO:4 of U.S. Patent U.S. Patent Publication no. 2005/0272680.

### Antibodies

As noted above, antibodies (including antigen-binding antibody fragments) specific for a lipid synthesis acyltransferase are suitable for use as a lipid synthesis acyltransferase inhibitor.

Methods of making antibodies specific for a lipid synthesis acyltransferase are known in the art. Briefly, suitable antibodies can be generated by immunizing a host animal with peptides comprising all or a portion of a lipid synthesis acyltransferase protein, such as DGAT1, DGAT2, ACAT1, or ACAT2. Suitable host animals include mouse, rat, sheep, goat, hamster, rabbit, etc. The origin of the protein immunogen can be mouse, human, rat, monkey, recombinant, etc. The host animal will generally be a different species than the immunogen.

Immunogens can comprise all or a part of a lipid synthesis acyltransferase protein, in which the protein can further comprise post-translational modification, natural or synthetic modifications. The antibody can be produced as a single chain or multimeric structure. DNA sequences encoding the variable region of the heavy chain and the variable region of the light chain can be ligated to a spacer to encode a protein that retains the specificity and the affinity of the antibody.

In some embodiments, the antibody is a humanized monoclonal antibody. Methods of humanizing antibodies are known in the art. The humanized antibody can be the product of an animal having transgenic human immunoglobulin constant region genes. See WO90/10077 and WO90/04036. Alternatively, the antibody can be engineered by recombinant DNA techniques to incorporate fragment work corresponding to the human sequence. See WO92/02190.

In some embodiments, the antibody is an antigen-binding antibody fragment. Antibody fragments, such as Fv, F(ab')₂ and Fab can be prepared by cleavage of the intact protein, e.g. by protease or chemical cleavage. Alternatively, a truncated gene encoding the antibody fragment is designed and is expressed in a suitable host cell to generate the encoded antibody fragment. For example, a chimeric gene encoding a portion of the F(ab')₂ fragment would include nucleotide sequences encoding the CH1 domain and hinge region of the H chain, followed by a translational stop codon to yield the truncated antibody.

In some embodiments, a suitable antibody is an "artificial" antibody, e.g., antibodies and antibody fragments produced and selected *in vitro.* In some embodiments, such antibodies are displayed on the surface of a bacteriophage or other viral particle. In some embodiments, such artificial antibodies are present as fusion proteins with a viral or bacteriophage structural protein, including, but not limited to, M13 gene III protein. Methods of producing such artificial antibodies are well known in the art. See, e.g., U.S. Patent Nos. 5,516,637; 5,223,409; 5,658,727; 5,667,988; 5,498,538; 5,403,484; 5,571,698; and 5,625,033.

### Measuring HCV viral load

Whether a subject method is effective in treating an HCV infection can be determined in various ways, including measuring HCV viral load in an individual being treated. Viral load can be measured by measuring the titer or level of virus in serum. These methods include, but are not limited to, a quantitative polymerase chain reaction (PCR) and a branched DNA (bDNA) test. Quantitative assays for measuring the viral load (titer) of HCV RNA have been developed. Many such assays are available commercially, including a quantitative reverse transcription PCR (RT-PCR) (Amplicor HCV Monitor™, Roche Molecular Systems, New Jersey); and a branched DNA (deoxyribonucleic acid) signal amplification assay (Quantiplex™ HCV RNA Assay (bDNA), Chiron Corp., Emeryville, California). See, e.g., Gretch et al. (1995) Ann. Intern. Med. 123:321-329. Also of interest is a nucleic acid test (NAT), developed by Gen-Probe Inc. (San Diego) and Chiron Corporation, and sold by Chiron Corporation under the trade name Procleix®, which NAT simultaneously tests for the presence of HIV-1 and HCV. See, e.g., Vargo et al. (2002) Transfusion 42:876-885.

### Methods of assessing liver function

Liver fibrosis reduction is determined by analyzing a liver biopsy sample. An analysis of a liver biopsy comprises assessments of two major components: necroinflammation assessed by "grade" as a measure of the severity and ongoing disease activity, and the lesions of fibrosis and parenchymal or vascular remodeling as assessed by "stage" as being reflective of long-term disease progression. See, e.g., Brunt (2000) Hepatol. 31:241-246; and METAVIR (1994) Hepatology 20:15-20. Based on analysis of the liver biopsy, a score is assigned. A number of standardized scoring systems exist which provide a quantitative assessment of the degree and severity of fibrosis. These include the METAVIR, Knodell, Scheuer, Ludwig, and Ishak scoring systems.

The METAVIR scoring system is based on an analysis of various features of a liver biopsy, including fibrosis (portal fibrosis, centrilobular fibrosis, and cirrhosis); necrosis (piecemeal and lobular necrosis, acidophilic retraction, and ballooning degeneration); inflammation (portal tract inflammation, portal lymphoid aggregates, and distribution of portal inflammation); bile duct changes; and the Knodell index (scores of periportal necrosis, lobular necrosis, portal inflammation, fibrosis, and overall disease activity). The definitions of each stage in the METAVIR system are as follows: score: 0, no fibrosis; score: 1, stellate enlargement of portal tract but without septa formation; score: 2, enlargement of portal tract with rare septa formation; score: 3, numerous septa without cirrhosis; and score: 4, cirrhosis.

Knodell's scoring system, also called the Hepatitis Activity Index, classifies specimens based on scores in four categories of histologic features: I. Periportal and/or budging necrosis; II. Intralobular degeneration and focal necrosis; III. Portal inflammation; and IV. Fibrosis. In the Knodell staging system, scores are as follows: score: 0, no fibrosis; score: 1, mild fibrosis (fibrous portal expansion); score: 2, moderate fibrosis; score: 3, severe fibrosis (bridging fibrosis); and score: 4, cirrhosis. The higher the score, the more severe the liver tissue damage. Knodell (1981) Hepatol. 1:431.

In the Scheuer scoring system scores are as follows: score: 0, no fibrosis; score: 1, enlarged, fibrotic portal tracts; score: 2, periportal or portal-portal septa, but intact architecture; score: 3, fibrosis with architectural distortion, but no obvious cirrhosis; score: 4, probable or definite cirrhosis. Scheuer (1991) J. Hepatol. 13:372.

The Ishak scoring system is described in Ishak (1995) J. Hepatol. 22:696-699. Stage 0, No fibrosis; Stage 1, Fibrous expansion of some portal areas, with or without short fibrous septa; stage 2, Fibrous expansion of most portal areas, with or without short fibrous septa; stage 3, Fibrous expansion of most portal areas with occasional portal to portal (P-P) bridging; stage 4, Fibrous expansion of portal areas with marked budging (P-P) as well as portal-central (P-C); stage 5, Marked budging (P-P and/or P-C) with occasional nodules (incomplete cirrhosis); stage 6, Cirrhosis, probable or definite.

The benefit of a subject therapy can also be measured and assessed by using the Child-Pugh scoring system which comprises a multicomponent point system based upon abnormalities in serum bilirubin level, serum albumin level, prothrombin time, the presence and severity of ascites, and the presence and severity of encephalopathy. Based upon the presence and severity of abnormality of these parameters, patients can be placed in one of three categories of increasing severity of clinical disease: A, B, or C.

### Combination therapy

In some embodiments, a subject method involves administering to an individual an effective amount of an active agent that reduces the level and/or activity of a lipid synthesis acyltransferase, in combination therapy with one or more additional therapeutic agents. Suitable additional therapeutic agents include agents suitable for treating an HCV infection, e.g., an interferon-alpha (IFN-α), a nucleoside analog, an HCV NS3 inhibitor, an HCV NS5B inhibitor, etc.

### Ribavirin

In some embodiments, the at least one additional suitable therapeutic agent includes ribavirin. Ribavirin, 1-β-D-ribofuranosyl-1H-1,2,4-triazole-3-carboxamide, available from ICN Pharmaceuticals, Inc., Costa Mesa, Calif., is described in the Merck Index, compound No. 8199, Eleventh Edition. Its manufacture and formulation is described in U.S. Pat. No. 4,211,771. The invention also contemplates use of derivatives of ribavirin (see, *e.g.,* U.S. Pat. No. 6,277,830). The ribavirin can be administered orally in capsule or tablet form, or in the same or different administration form and in the same or different route as the lipid synthesis acyltransferase inhibitor. Of course, other types of administration of both medicaments, as they become available are contemplated, such as by nasal spray, transdermally, by suppository, by sustained release dosage form, etc. Any form of administration is suitable so long as the proper dosages are delivered without destroying the active ingredient.

Ribavirin is generally administered in an amount ranging from about 400 mg to about 1200 mg, from about 600 mg to about 1000 mg, or from about 700 to about 900 mg per day. In some embodiments, ribavirin is administered throughout the entire course of lipid synthesis acyltransferase inhibitor therapy. In other embodiments, ribavirin is administered only during the first period of time. In still other embodiments, ribavirin is administered only during the second period of time.

### Levovirin

In some embodiments, the at least one additional suitable therapeutic agent includes levovirin. Levovirin is the L-enantiomer of ribavirin, and exhibits the property of enhancing a Th1 immune response over a Th2 immune response. Levovirin is manufactured by ICN Pharmaceuticals.

Levovirin has the following structure:

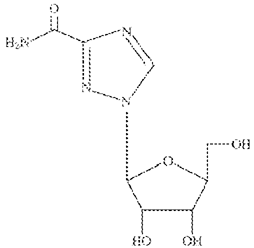

### Viramidine

In some embodiments, the at least one additional suitable therapeutic agent includes viramidine. Viramidine is a 3-carboxamidine derivative of ribavirin, and acts as a prodrug of ribavirin. It is efficiently converted to ribavirin by adenosine deaminases.

Viramidine has the following structure:

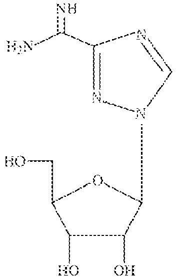

### Nucleoside analogs

Nucleoside analogs that are suitable for use in a subject treatment method include, but are not limited to, ribavirin, levovirin, viramidine, isatoribine, an L-ribofuranosyl nucleoside as disclosed in U.S. Patent No. 5,559,101 and encompassed by Formula I of U.S. Patent No. 5,559,101 (e.g., 1-β-L-ribofuranosyluracil, 1-β-L-ribofuranosyl-5-fluorouracil, 1-β-L-ribofuranosylcytosine, 9-βL-ribofuranosyladenine, 9-β-L-ribofuranosylhypoxanthine, 9-β-L-ribofuranosylguanine, 9-β-L-ribofuranosyl-6-thioguanine, 2-amino-α-L-ribofuran1[1',2':4,5]oxazoline, O²,O²-anhydro-1-α-L-ribofuranosyluracil, 1-α-L-ribofuranosyluracil, 1-(2,3,5-tri-O-benzoyl-α-ribofuranosyl)-4-thiouracil, 1-α-L-ribofuranosylcytosine, 1-α-L-ribofuranosyl-4-thiouracil, 1-α-L-ribofuranosyl-5-fluorouracil, 2-amino-β-L-arabinofurano[1',2':4,5]oxazoline, O²,O²-anhydro-β-L-arabinofuranosyluracil, 2'-deoxy-β-L-uridine, 3'5'-Di-O-benzoyl-2'deoxy-4-thio β-L-uridine, 2'-deoxy-β-L-cytidine, 2'-deoxy-β-L-4-thiouridine, 2'-deoxy-β-L-thymidine, 2'-deoxy-β-L-5-fluorouridine, 2',3'-dideoxy-β-L-uridine, 2'-deoxy-β-L-5-fluorouridine, and 2'-deoxy-β-L-inosine); a compound as disclosed in U.S. Patent No. 6,423,695 and encompassed by Formula I of U.S. Patent No. 6,423,695; a compound as disclosed in U.S. Patent Publication No. 2002/0058635, and encompassed by Formula 1 of U.S. Patent Publication No. 2002/0058635; a nucleoside analog as disclosed in WO 01/90121 A2 (Idenix); a nucleoside analog as disclosed in WO 02/069903 A2 (Biocryst Pharmaceuticals Inc.); a nucleoside analog as disclosed in WO 02/057287 A2 or WO 02/057425 A2 (both Merck/Isis); and the like.

### HCV NS3 inhibitors

In some embodiments, the at least one additional suitable therapeutic agent includes HCV NS3 inhibitors. Suitable HCV non-structural protein-3 (NS3) inhibitors include, but are not limited to, a tri-peptide as disclosed in U.S. Patent Nos. 6,642,204, 6,534,523, 6,420,380, 6,410,531, 6,329,417, 6,329,379, and 6,323,180 (Boehringer-Ingelheim); a compound as disclosed in U.S. Patent No. 6,143,715 (Boehringer-Ingelheim); a macrocyclic compound as disclosed in U.S. Patent no. 6,608,027 (Boehringer-Ingelheim); an NS3 inhibitor as disclosed in U.S. Patent Nos. 6,617,309, 6,608,067, and 6,265,380 (Vertex Pharmaceuticals); an azapeptide compound as disclosed in U.S. Patent No. 6,624,290 (Schering); a compound as disclosed in U.S. Patent No. 5,990,276 (Schering); a compound as disclosed in Pause et al. (2003) J. Biol. Chem. 278:20374-20380; NS3 inhibitor BILN 2061 (Boehringer-Ingelheim; Lamarre et al. (2002) Hepatology 36:301A; and Lamarre et al. (Oct. 26, 2003) Nature doi:10.1038/nature02099); NS3 inhibitor VX-950 (Vertex Pharmaceuticals; Kwong et al. (Oct. 24-28, 2003) 54^{th} Ann. Meeting AASLD); NS3 inhibitor SCH6 (Abib et al. (October 24-28, 2003) Abstract 137. Program and Abstracts of the 54^{th} Annual Meeting of the American Association for the Study of Liver Diseases (AASLD). October 24-28, 2003. Boston, MA.); any of the NS3 protease inhibitors disclosed in WO 99/07733, WO 99/07734, WO 00/09558, WO 00/09543, WO 00/59929 or WO 02/060926 (e.g., compounds 2, 3, 5, 6, 8, 10, 11, 18, 19, 29, 30, 31, 32, 33, 37, 38, 55, 59, 71, 91, 103, 104, 105, 112, 113, 114, 115, 116, 120, 122, 123, 124, 125, 126 and 127 disclosed in the table of pages 224-226 in WO 02/060926); an NS3 protease inhibitor as disclosed in any one of U.S. Patent Publication Nos. 2003019067, 20030187018, 20030186895, 2007/0054842, and 2008/0019942; and the like.

Of particular interest in many embodiments are NS3 inhibitors that are specific NS3 inhibitors, e.g., NS3 inhibitors that inhibit NS3 serine protease activity and that do not show significant inhibitory activity against other serine proteases such as human leukocyte elastase, porcine pancreatic elastase, or bovine pancreatic chymotrypsin, or cysteine proteases such as human liver cathepsin B.

### NS5B inhibitors

In some embodiments, the at least one additional suitable therapeutic agent includes NS5B inhibitors. Suitable HCV non-structural protein-5 (NS5; RNA-dependent RNA polymerase) inhibitors include, but are not limited to, a compound as disclosed in U.S. Patent No. 6,479,508 (Boehringer-Ingelheim); a compound as disclosed in any of International Patent Application Nos. PCT/CA02/01127, PCT/CA02/01128, and PCT/CA02/01129, all filed on July 18, 2002 by Boehringer Ingelheim; a compound as disclosed in U.S. Patent No. 6,440,985 (ViroPharma); a compound as disclosed in WO 01/47883, e.g., JTK-003 (Japan Tobacco); a dinucleotide analog as disclosed in Zhong et al. (2003) Antimicrob. Agents Chemother. 47:2674-2681; a benzothiadiazine compound as disclosed in Dhanak et al. (2002) J. Biol Chem. 277(41):38322-7; an NS5B inhibitor as disclosed in WO 02/100846 A1 or WO 02/100851 A2 (both Shire); an NS5B inhibitor as disclosed in WO 01/85172 A1 or WO 02/098424 A1 (both Glaxo SmithKline); an NS5B inhibitor as disclosed in WO 00/06529 or WO 02/06246 A1 (both Merck); an NS5B inhibitor as disclosed in WO 03/000254 (Japan Tobacco); an NS5B inhibitor as disclosed in EP 1 256,628 A2 (Agouron); JTK-002 (Japan Tobacco); JTK-109 (Japan Tobacco); and the like.

Of particular interest in many embodiments are NS5 inhibitors that are specific NS5 inhibitors, e.g., NS5 inhibitors that inhibit NS5 RNA-dependent RNA polymerase and that lack significant inhibitory effects toward other RNA dependent RNA polymerases and toward DNA dependent RNA polymerases.

### Interferon-alpha

In some embodiments, the at least one additional suitable therapeutic agent includes an IFN-α. Any known IFN-α can be used in the instant invention. The term "interferon-alpha" as used herein refers to a family of related polypeptides that inhibit viral replication and cellular proliferation and modulate immune response. The term " IFN-α " includes naturally occurring IFN-α; synthetic IFN-α; derivatized IFN-α (e.g., PEGylated IFN-α, glycosylated IFN-α, and the like); and analogs of naturally occurring or synthetic IFN-α; essentially any IFN-α that has antiviral properties, as described for naturally occurring IFN-α.

Suitable alpha interferons include, but are not limited to, naturally-occurring IFN-α (including, but not limited to, naturally occurring IFN-α2a, IFN-α2b); recombinant interferon alpha-2b such as Intron-A interferon available from Schering Corporation, Kenilworth, N.J.; recombinant interferon alpha-2a such as Roferon interferon available from Hoffmann-La Roche, Nutley, N.J.; recombinant interferon alpha-2C such as Berofor alpha 2 interferon available from Boehringer Ingelheim Pharmaceutical, Inc., Ridgefield, Conn.; interferon alpha-n1, a purified blend of natural alpha interferons such as Sumiferon available from Sumitomo, Japan or as Weliferon interferon alpha-n1 (INS) available from the Glaxo-Wellcome Ltd., London, Great Britain; and interferon alpha-n3 a mixture of natural alpha interferons made by Interferon Sciences and available from the Purdue Frederick Co., Norwalk, Conn., under the Alferon Tradename.

The term "IFN-α" also encompasses consensus IFN-α. Consensus IFN-α (also referred to as "CIFN" and "IFN-con" and "consensus interferon") encompasses but is not limited to the amino acid sequences designated IFN-con₁, IFN-con₂ and IFN-con₃ which are disclosed in U.S. Pat. Nos. 4,695,623 and 4,897,471; and consensus interferon as defined by determination of a consensus sequence of naturally occurring interferon alphas (e.g., INFERGEN™), InterMune, Inc., Brisbane, Calif.). IFN-con₁ is the consensus interferon agent in the INFERGEN™ alfacon-1 product. The INFERGEN™ consensus interferon product is referred to herein by its brand name (INFERGEN™) or by its generic name (interferon alfacon-1). DNA sequences encoding IFN-con can be synthesized as described in the aforementioned patents or other standard methods.

Also suitable for use in a subject treatment method are fusion polypeptides comprising an IFN-α and a heterologous polypeptide. Suitable IFN-α fusion polypeptides include, but are not limited to, Albuferon-alpha™ (a fusion product of human albumin and IFN-α; Human Genome Sciences; see, e.g., Osborn et al. (2002) J. Pharmacol. Exp. Therap. 303:540-548). Also suitable for use in a subject treatment method are gene-shuffled forms of IFN-α. See, e.g., Masci et al. (2003) Curr. Oncol. Rep. 5:108-113.

The term "IFN-α" also encompasses derivatives of IFN-α that are derivatized (e.g., are chemically modified) to alter certain properties such as serum half-life. As such, the term " IFN-α" includes glycosylated IFN-α; IFN-α derivatized with poly(ethylene glycol) ("PEGylated IFN-α "); and the like. PEGylated IFN-α, and methods for making same, is discussed in, e.g., U.S. Pat. Nos. 5,382,657; 5,981,709; and 5,951,974. PEGylated IFN-α encompasses conjugates of PEG and any of the above-described IFN-α molecules, including, but not limited to, PEG conjugated to interferon alpha-2a (Roferon, Hoffman La-Roche, Nutley, N.J.), interferon alpha 2b (Intron, Schering-Plough, Madison, N.J.), interferon alpha-2c (Berofor Alpha, Boebringer Ingelheim, Ingelheim, Germany); and consensus interferon as defined by determination of a consensus sequence of naturally occurring interferon alphas (INFERGEN™, InterMune, Inc., Brisbane, Calif.).

### FORMULATIONS, DOSAGES, ROUTES OF ADMINISTRATION

An active agent (an agent that reduces the level and/or activity of a lipid synthesis acyltransferase and optionally one or more additional therapeutic agents) is administered to individuals in a formulation with a pharmaceutically acceptable excipient(s). A wide variety of pharmaceutically acceptable excipients are known in the art and need not be discussed in detail herein. Pharmaceutically acceptable excipients have been amply described in a variety of publications, including, for example, A. Gennaro (2000) "Remington: The Science and Practice of Pharmacy," 20th edition, Lippincott, Williams, & Wilkins; Pharmaceutical Dosage Forms and Drug Delivery Systems (1999) H. C. Ansel et al., eds., 7th ed., Lippincott, Williams, & Wilkins; and Handbook of Pharmaceutical Excipients (2000) A. H. Kibbe et al., eds., 3rd ed. Amer. Pharmaceutical Assoc.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

In a subject treatment method, an active agent (an agent that reduces the level and/or activity of a lipid synthesis acyltransferase; and optionally one or more additional active agents) can be administered to an individual in need thereof using any convenient means capable of resulting in the desired therapeutic effect. Thus, the agents can be incorporated into a variety of formulations for therapeutic administration. More particularly, an active agent (an agent that reduces the level and/or activity of a lipid synthesis acyltransferase; and optionally one or more additional active agents) can be formulated into pharmaceutical compositions by combination with appropriate, pharmaceutically acceptable carriers or diluents, and can be formulated into preparations in solid, semi-solid, liquid or gaseous forms, such as tablets, capsules, powders, granules, ointments, solutions, suppositories, injections, inhalants and aerosols.

As such, administration of an active agent (an agent that reduces the level and/or activity of a lipid synthesis acyltransferase; and optionally one or more additional active agents) can be achieved in various ways, including oral, buccal, rectal, parenteral, intraperitoneal, intradermal, subcutaneous, intramuscular, transdermal, intratracheal, etc., administration. In some embodiments, two different routes of administration are used. As one non-limiting example, a DGAT1 inhibitor is administered orally; IFN-α is administered subcutaneously by injection; and ribavirin is administered orally.

Subcutaneous administration of an active agent (an agent that reduces the level and/or activity of a lipid synthesis acyltransferase; and optionally one or more additional active agents) can be accomplished using standard methods and devices, e.g., needle and syringe, a subcutaneous injection port delivery system, and the like. See, e.g., U.S. Pat. Nos. 3,547,119; 4,755,173; 4,531,937; 4,311,137; and 6,017,328. A combination of a subcutaneous injection port and a device for administration of a therapeutic agent to a patient through the port is referred to herein as "a subcutaneous injection port delivery system." In some embodiments, subcutaneous administration is achieved by a combination of devices, e.g., bolus delivery by needle and syringe, followed by delivery using a continuous delivery system.

In some embodiments, a therapeutic agent (an agent that reduces the level and/or activity of a lipid synthesis acyltransferase; and optionally one or more additional active agents) is delivered by a continuous delivery system. The term "continuous delivery system" is used interchangeably herein with "controlled delivery system" and encompasses continuous (e.g., controlled) delivery devices (e.g., pumps) in combination with catheters, injection devices, and the like, a wide variety of which are known in the art.

Mechanical or electromechanical infusion pumps can also be suitable for use with a subject treatment method. Examples of such devices include those described in, for example, U.S. Pat. Nos. 4,692,147; 4,360,019; 4,487,603; 4,360,019; 4,725,852; 5,820,589; 5,643,207; 6,198,966; and the like. In general, the present methods of drug delivery can be accomplished using any of a variety of refillable, pump systems. Pumps provide consistent, controlled release over time. Typically, the agent is in a liquid formulation in a drug-impermeable reservoir, and is delivered in a continuous fashion to the individual.

In one embodiment, the drug delivery system is an at least partially implantable device. The implantable device can be implanted at any suitable implantation site using methods and devices well known in the art. An implantation site is a site within the body of a subject at which a drug delivery device is introduced and positioned. Implantation sites include, but are not necessarily limited to a subdermal, subcutaneous, intramuscular, or other suitable site within a subject's body. Subcutaneous implantation sites are used in some embodiments because of convenience in implantation and removal of the drug delivery device.

Drug release devices suitable for use in the invention can be based on any of a variety of modes of operation. For example, the drug release device can be based upon a diffusive system, a convective system, or an erodible system (e.g., an erosion-based system). For example, the drug release device can be an electrochemical pump, osmotic pump, an electroosmotic pump, a vapor pressure pump, or osmotic bursting matrix, e.g., where the drug is incorporated into a polymer and the polymer provides for release of drug formulation concomitant with degradation of a drug-impregnated polymeric material (e.g., a biodegradable, drug-impregnated polymeric material). In other embodiments, the drug release device is based upon an electrodiffusion system, an electrolytic pump, an effervescent pump, a piezoelectric pump, a hydrolytic system, etc.

Drug release devices based upon a mechanical or electromechanical infusion pump can also be suitable for use with a subject treatment method. Examples of such devices include those described in, for example, U.S. Pat. Nos. 4,692,147; 4,360,019; 4,487,603; 4,360,019; 4,725,852, and the like. In general, a subject treatment method can be accomplished using any of a variety of refillable, non-exchangeable pump systems. Pumps and other convective systems will in some embodiments be used, due to their generally more consistent, controlled release over time. Osmotic pumps are particularly preferred due to their combined advantages of more consistent controlled release and relatively small size (see, e.g., PCT published application no. WO 97/27840 and U.S. Pat. Nos. 5,985,305 and 5,728,396)). Exemplary osmotically-driven devices suitable for use in the invention include, but are not necessarily limited to, those described in U.S. Pat. Nos. 3,760,984; 3,845,770; 3,916,899; 3,923,426; 3,987,790; 3,995,631; 3,916,899; 4,016,880; 4,036,228; 4,111,202; 4,111,203; 4,203,440; 4,203,442; 4,210,139; 4,327,725; 4,627,850; 4,865,845; 5,057,318; 5,059,423; 5,112,614; 5,137,727; 5,234,692; 5,234,693; 5,728,396; and the like.

In pharmaceutical dosage forms, the active agent(s) is administered in the form of its pharmaceutically acceptable salts, or the active agent is used alone or in appropriate association, as well as in combination, with other pharmaceutically active compounds. The following methods and excipients are merely exemplary and are in no way limiting.

For oral preparations, an active agent can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

An active agent can be formulated into preparations for injection by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

Furthermore, the active agents can be made into suppositories by mixing with a variety of bases such as emulsifying bases or water-soluble bases. An active agent can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions can be provided wherein each dosage unit, for example, teaspoonful, tablespoonful, tablet or suppository, contains a predetermined amount of the composition containing one or more inhibitors. Similarly, unit dosage forms for injection or intravenous administration can comprise the active agent(s) in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of an active agent calculated in an amount sufficient to produce the desired effect in association with a pharmaceutically acceptable diluent, carrier or vehicle. The specifications for a particular active agent depend on the particular agent employed and the effect to be achieved, and the pharmacodynamics associated with each agent in the host.

In connection with each of the methods described herein, the invention provides embodiments in which the therapeutic agent(s) is/are administered to the patient by a controlled drug delivery device. In some embodiments, the therapeutic agent(s) is/are delivered to the patient substantially continuously or continuously by the controlled drug delivery device. Optionally, an implantable infusion pump is used to deliver the therapeutic agent(s) to the patient substantially continuously or continuously by subcutaneous infusion. In other embodiments, a therapeutic agent is administered to the patient so as to achieve and maintain a desired average daily serum concentration of the therapeutic agent at a substantially steady state for the duration of the monotherapy or combination therapy. Optionally, an implantable infusion pump is used to deliver the therapeutic agent to the patient by subcutaneous infusion so as to achieve and maintain a desired average daily serum concentration of the therapeutic agent at a substantially steady state for the duration of the therapeutic agent in monotherapy or combination therapy.

### SUBJECTS SUITABLE FOR TREATMENT

Individuals who are to be treated according to a subject treatment method include individuals who have been clinically diagnosed as infected with HCV. Individuals who are infected with HCV are identified as having HCV RNA in their blood, and/or having anti-HCV antibody in their serum.

In particular embodiments of interest, individuals have an HCV titer of at least about 10⁵, at least about 5 x 10⁵, or at least about 10⁶, or at least about 2 x 10⁶, genome copies of HCV per milliliter of serum. The patient may be infected with any HCV genotype (genotype 1, including 1a and 1b, 2, 3, 4, 6, etc. and subtypes (e.g., 2a, 2b, 3a, etc.)), e.g., a difficult to treat genotype such as HCV genotype 1, or particular HCV subtypes and quasispecies. In some embodiments, the individual is infected with HCV genotype 1. In some embodiments, the individual is infected with HCV genotype 1b.

Also of interest are HCV-positive individuals who exhibit severe fibrosis or early cirrhosis (non-decompensated, Child's-Pugh class A or less), or more advanced cirrhosis (decompensated, Child's-Pugh class B or C) due to chronic HCV infection. In particular embodiments of interest, HCV-positive individuals with stage 3 or 4 liver fibrosis according to the METAVIR scoring system are suitable for treatment with a subject treatment method. In other embodiments, individuals suitable for treatment with a subject treatment method are patients with decompensated cirrhosis with clinical manifestations, including patients with far-advanced liver cirrhosis, including those awaiting liver transplantation. In still other embodiments, individuals suitable for treatment with the methods of the instant invention include patients with milder degrees of fibrosis including those with early fibrosis (stages 1 and 2 in the METAVIR, Ludwig, and Scheuer scoring systems; or stages 1, 2, or 3 in the Ishak scoring system.).

Individuals who are clinically diagnosed as infected with HCV include naive individuals (e.g., individuals not previously treated for HCV) and individuals who have failed prior treatment for HCV ("treatment failure" patients).

The term "treatment failure patients" (or "treatment failures") as used herein generally refers to HCV-infected patients who failed to respond to previous therapy for HCV (referred to as "non-responders") or who initially responded to previous therapy, but in whom the therapeutic response was not maintained (referred to as "relapsers").

Patients suitable for treatment with a subject treatment method include treatment failure patients, which include patients who failed to respond to previous HCV therapy (referred to as "non-responders") or who initially responded to previous therapy, but in whom the therapeutic response was not maintained (referred to as "relapsers"). As non-limiting examples, individuals may have an HCV titer of at least about 10⁵, at least about 5 x 10⁵, or at least about 10⁶, genome copies of HCV per milliliter of serum.

Individuals who are to be treated with a subject method for treating an HCV infection include individuals who have been clinically diagnosed as infected with HCV. Individuals who are infected with HCV are identified as having HCV RNA in their blood, and/or having anti-HCV antibody in their serum.

In some embodiments, an individual to be treated according to a subject treatment method is an individual who has liver steatosis and who is HCV infected. In some embodiments, an individual to be treated according to a subject treatment method is an individual who has liver fibrosis and who is HCV infected.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric. Standard abbreviations may be used, e.g., bp, base pair(s); kb, kilobase(s); pl, picoliter(s); s or sec, second(s); min, minute(s); h or hr, hour(s); aa, amino acid(s); kb, kilobase(s); bp, base pair(s); nt, nucleotide(s); i.m., intramuscular(ly); i.p., intraperitoneal(ly); s.c., subcutaneous(ly); and the like. "α-X" refers to an antibody to X; e.g., "α-Core" refers to an antibody that binds Core.

### Example 1: Effect of DGAT1 inhibitor on HCV core-induced lipid droplet formation Core-induced lipid droplet accumulation depended on DGAT1.

To study Hepatitis C Virus (HCV) core-induced lipid droplet formation, the murine fibroblast cell line NIH3T3 was used, as these cells have low lipid droplet content. Upon expression of HCV core ("core") via a lentiviral construct expressing core, NIH3T3 cells strongly accumulate lipid droplets as shown by Oil-red-O (ORO) staining. To inhibit DGAT1, a small molecule inhibitor that specifically inhibits DGAT1 activity with an IC₅₀ of 300 nM was used. The DGAT1 inhibitor that was used has the chemical name: 2-((1 s,4s)-4-(4-(4-amino-7,7-dimethyl-7H-pyrimido[4,5-b][1,4]oxazin-6-yl)phenyl)cyclohexyl)acetic acid; and the following structure:

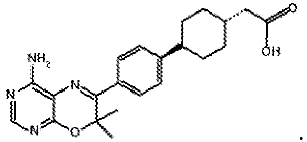

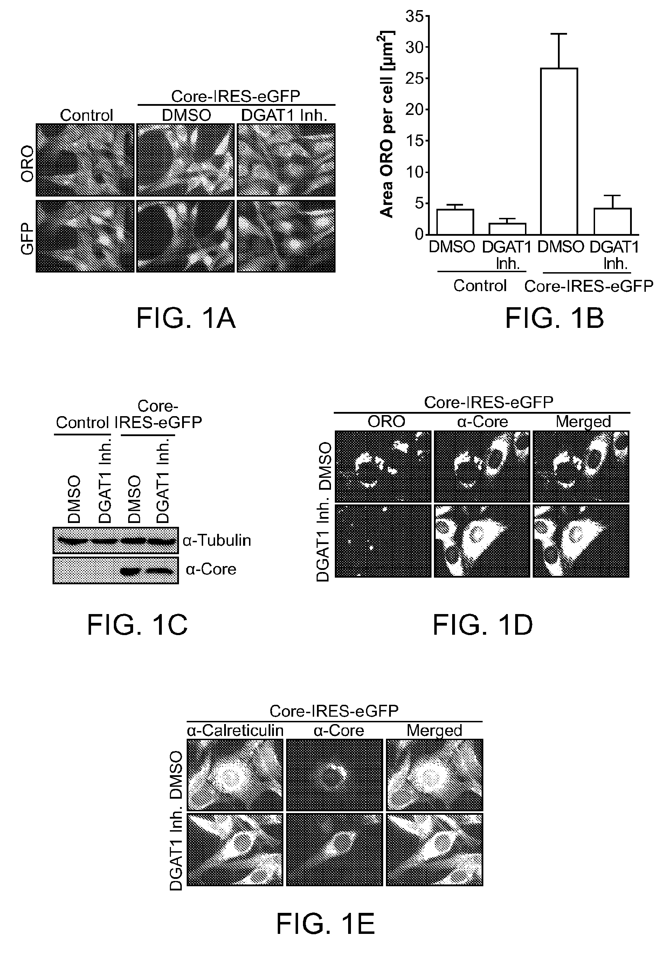

Treatment of Core-expressing NIH3T3 cells with 20 µM DGAT1 inhibitor completely suppresses lipid droplet accumulation (**Figure 1A**). Quantification of these images revealed that Core expression leads to a five fold increase in lipid droplet area per cell, which was completely blocked by DGAT1 inhibition (**Figure 1B**).

To exclude any effects of DGAT1 inhibition on Core stability, expression levels in treated and untreated cells were verified by immunoblot using Core specific antibodies. Core expression was stable in DGAT1 inhibitor treated cells (**Figure 1C**). Core strongly localizes to lipid droplets in NIH3T3 cells. Immunostaining was performed using an anti-core ("α-Core") antibody on Core-expressing NIH3T3 cells incubated in the presence or absence of the DGAT1 inhibitor. Cells were subsequently treated with ORO to stain the lipid droplets. As shown in **Figure 1D**, Core localizes to the lipid droplets in control treated cells. However, when lipid droplet formation is blocked by DGAT1 inhibition, Core is retained at the ER, where it is translated. A co-immunostaining with an endoplasmic reticulum (ER) marker protein (Calreticulin) showed complete co-localization of Core with the ER marker in DGAT1 inhibitor treated cells. Interference with Core-induced lipid droplet formation therefore alters the subcellular localization of the Core protein.

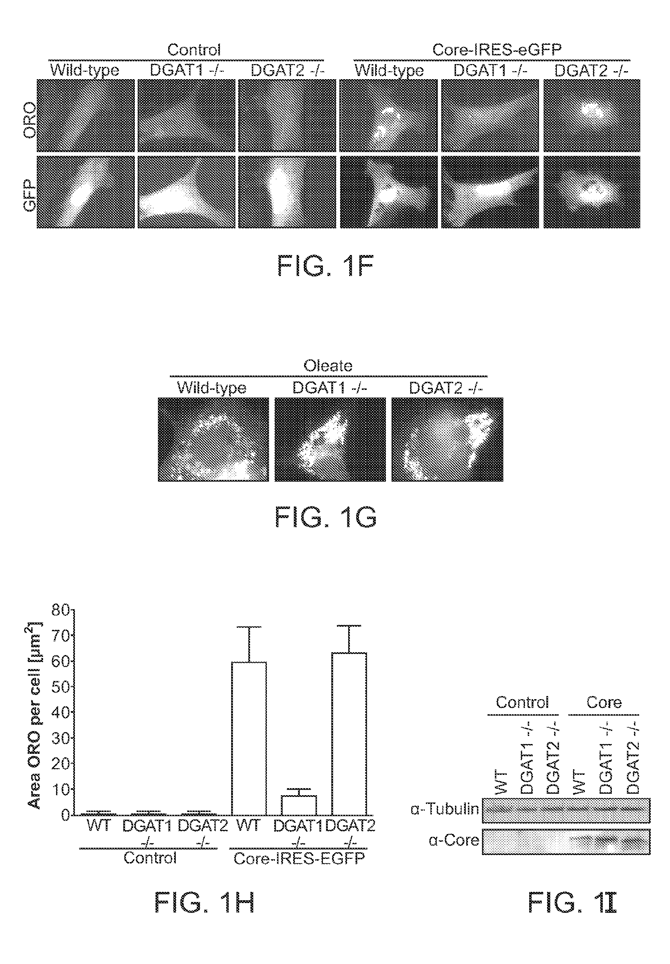

To confirm that Core specifically utilizes DGAT1 to induce lipid droplets, the experiments described above were performed in mouse embryonic fibroblasts (MEFs) from wild-type mice, DGAT1 knockout (DGAT1^{-/-}) mice, and DGAT2 knockout (DGAT2^{-/-}) mice. Wild type, DGAT1 -/- and DGAT2 -/- MEFs were transduced with a Core-expressing lentivirus. Transduced cells were stained with ORO to visualize lipid droplets and analyzed by epifluorescence microscopy. As shown in **Figure 1F****,** core expression leads to an accumulation of lipid droplets compared to control cells in wild-type MEFs. In stark contrast, Core-induced lipid droplet accumulation is strongly attenuated in DGAT1 -/- MEFs but not in DGAT2 -/-MEFs, although the Core protein was equally expressed in all three cell types as shown by immunoblot (**Figure 1I****)**. A quantification of the ORO positive area showed that lipid droplet accumulation was nearly completely suppressed in DGAT1-/- MEFs (**Figure 1H**). Interestingly, DGAT1 -/- MEFs are able to form lipid droplets upon loading with oleate (**Figure 1G**), which suggests that the observed suppressive effect is not due to a complete defect in neutral lipid biosynthesis. The results indicate that Core requires DGAT1 to induce lipid droplet accumulation.

**Figures 1A-I.** For Figures A-D, NIH3T3 cells were transduced with a lentiviral vector expressing either eGFP (Control) or HCV Core-IRES-eGFP (HCV Core-internal ribosome entry site-enhanced green fluorescent protein), treated with dimethylsulfoxide (DMSO) or 20 µM DGAT1 inhibitor (day 1), fixed and stained or lysed for sodium dodecyl sulfate-polyacrylamide gel electrophoresis (SDS-PAGE) (day 3). **A.** ORO staining to visualize lipid droplets**.B.** Quantification of A. Data were obtained by quantification of at least 500 cells. Error bars represent S.E.M. **C.** Immunoblot with α-Core and α-Tubulin antibodies. **D.** Immunofluorescence staining with α-Core antibody followed by Alexa 647-labelled α-mouse antibody and subsequent ORO staining. **E.** Immunofluorescence staining with α-Core and α-Calreticulin antibodies followed by Alexa 647-labelled α-mouse and Cy3-labelled α-rabbit antibodies. **F-I.** Wild-type, DGAT1-/-, and DGAT2 -/- mouse embryonic fibroblast (MEF) cells were transduced with a lentiviral vector expressing either enhanced green fluorescent protein (eGFP) (Control) or HCV Core-IRES-eGFP (day 0), treated with dimethylsulfoxide (DMSO) or 20 µM DGAT1 inhibitor (day 1), fixed and stained or lysed for SDS-PAGE (day 3). **F.** ORO staining to visualize lipid droplets. **G.** Wild-type, DGAT1-/-, and DGAT2-/- MEF cells were loaded with oleate for 24 h, fixed and lipid droplets were visualized by ORO staining. **H.** Quantification of F. Data was obtained by quantification of at least 50 cells. Error bars represent S.E.M. **E.** Immunoblot with α-Core and α-Tubulin antibodies.

### Example 2: HCV core expression delays triglyceride breakdown.

### Method

The measurement of lipolysis was performed as previously described (Brasaemle et al. (2000) J. Biol. Chem. 275:38486). Cells were incubated with 400 µM bovine serum albumin (BSA)-bound oleate containing 0.125 µCi/ml of [1-¹⁴C] oleic acid (GE Healthcare, 58 mCi/mmol) for 16 h to stimulate storage of triglycerides. Cells were then washed and incubated in fresh media containing 6 µM triacsin C for indicated times and the remaining cellular triglyceride determined as above. For microscopic analysis, cells were loaded with 400 µM BSA-bound oleate for 16 h, and then incubated in fresh medium in the presence of 6 µM triacsin C, fixed in paraformaldehyde (PFA) and stained with ORO and Hoechst.

### Results

It was speculated that core could stimulate triglyceride production by enhancing DGAT1 activity. However, no difference in *in vitro* DGAT1 activity was detected in lysates from Huh7 hepatoma cells expressing core or control cells, while addition of the DGAT1 inhibitor efficiently suppressed the activity (**Fig**. **2A**). Similarly, cellular triglyceride synthesis rates did not change when core was introduced into Huh7 cells (**Fig**. **2B**), human embryonic kidney 293 cells or NIH/3T3 fibroblasts.

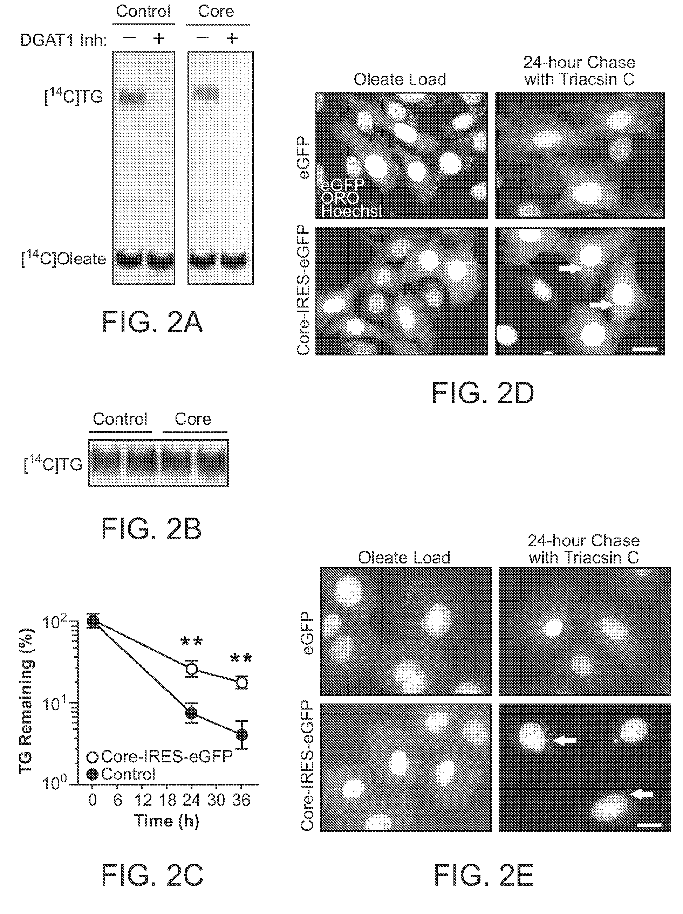

Next, it was tested whether lipid droplet breakdown was affected by core. In adipocytes, binding of perilipin to lipid droplets effectively prevents access of hormone-sensitive lipase and delays lipolysis in NIH/3T3 fibroblasts (Brasaemle et al. (2000) *supra*). The same experimental setup was used to test whether core has a 'stabilizing' effect on lipid droplets. Droplet formation was induced to equivalent levels in core-expressing and control cells by addition of oleate to the culture medium. After oleate removal, re-esterification of released fatty acids was inhibited by treatment with triacsin C (*N*-(((2*E*,4*E*,7*E*)-undeca-2,4,7-trienylidene)amino)nitrous amide), and cellular triglyceride content was measured by thin layer chromatography. While in control-transduced cells triglyceride levels decreased rapidly, core expression significantly preserved cellular triglyceride content (**Fig. 2C**). Groups of lipid droplets were visible in core-expressing (GFP-positive) cells after oil-red-O staining, while no lipid droplets were any longer detected in neighboring uninfected (GFP-negative) or control-transduced cells (**Fig. 2D**).

The same was observed in Huh7 hepatoma cells expressing core (**Fig. 2E**). It is important to note that hepatoma cells harbor three times more lipid droplets under regular culture conditions than are induced in fibroblasts after core expression. The overall increase in lipid droplet content in hepatoma cells is therefore modest in response to core expression or infection with HCV. However, our data unambiguously show that core expressed in hepatoma cells stabilizes a subset of lipid droplets by uncoupling them from the natural turnover of triglycerides.

**Figures 2A-E**. **A**. *In vitro* DGAT activity assays of cell lysates prepared from Huh7 cells transduced with lentiviral vectors expressing eGFP (control) or core-IRES-eGFP (core). Assays were performed in the presence or absence of the DGAT1 inhibitor. Extracted lipids were loaded on a thin layer chromatography plate and analyzed by autoradiography. **B.** Huh7 cells transduced with lentiviral vectors expressing eGFP (control) or core-IRES-eGFP (core) were incubated with radiolabelled oleate to quantify triglyceride synthesis *in vivo.* Lipid quantification was performed as in (A). **C.** Triglyceride turnover assay in NIH/3T3 cells transduced with lentiviral vectors expressing eGFP (control) or core-IRES-eGFP (core). Cells were loaded with radiolabelled oleate, washed and 'chased' in regular media containing triacsin C to inhibit re-esterification of released fatty acids. Extracted lipids were examined by thin layer chromatography and quantified using Bioscan (mean ± s.d.; n = 6; **p < 0.01). **D-E.** Epifluorescence microscopy of NIH/3T3 (D) or Huh7 cells (E) transduced with lentiviral vectors expressing eGFP or core-IRES-eGFP, loaded with oleate and 'chased' in the presence of triacsin C for 24 h. Cells were stained with ORO and Hoechst. Arrows mark stabilized lipid droplets that are protected from lipolysis. (scale bars = 20 µm).

### Example 3: Core and DGAT2 interact transiently in the ER

### Method

For immunoprecipitation experiments cells were lysed in lysis-buffer (150 mM NaCl, 1% NP-40, 1 mM EDTA, 50 mM Tris HCl, pH 7.4 and protease inhibitor cocktail (Sigma)) for 30 min and passed 10 times through a G23 needle. Clarified lysates were immunoprecipitated with antibody, specific to flagellin (FLAG) antigen and bound to agarose (α-FLAG M2 agarose) (Sigma; Bruzzard et al. (1994) BioTechniques 16:730) or DGAT1-specific antibody and protein A agarose (Invitrogen), washed 5 times in lysis buffer and resuspended in Laemmli buffer for sodium dodecyl sulfate-polyacrylamide gel electrophoresis (SDS-PAGE).

### Results

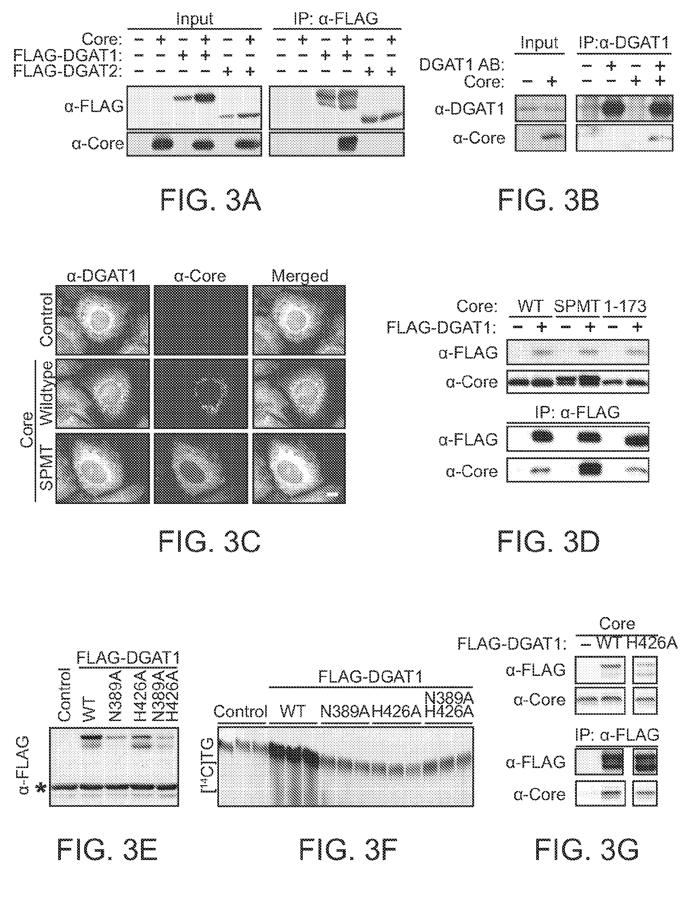

To study how DGAT1 affects these core functions, co-immunoprecipitation experiments were performed. Core co-immunoprecipitated with FLAG-DGAT1, but not FLAG-DGAT2, in 293T cells (**Fig. 3A**) and interacted with endogenous DGAT1 in Huh7 cells (**Fig. 3B**). Endogenous DGAT1 showed a reticular localization in Huh7 cells consistent with previous findings that it localizes to the ER. Upon core expression, DGAT1 was found in areas close to core-coated lipid droplets where it partially overlapped with core-generated signals (**Fig. 3C**).

Since DGAT1 itself is not considered a lipid droplet-associated protein, it was speculated core might interact with DGAT1 in the ER before migrating to newly synthesized lipid droplets. In support of this model, it was found endogenous DGAT1 effectively colocalized with a core mutant (SPMT) (McLauchlan et al. (2002) EMBO J. 21:3980) that is not fully processed at the signal peptide and resides in the ER (**Fig. 3C**). Indeed, FLAG-DGAT1 interacted stronger with the ER-based mutant than with wildtype core (**Fig. 3D**). Interaction with DGAT1 was not affected when a truncated form of core (amino acids 1-173) was examined excluding that the C-terminal membrane anchor of core is directly involved in the interaction (**Fig**. **3D**).

To test whether the catalytic activity of DGAT1 is required for the interaction with core, two point mutations (N389A and H426A) were introduced into the predicted catalytic domain of DGAT1, which were identified based on alignments with structurally related enzymes. The H426A mutant was stably expressed after transfection (**Fig**. **3E**) and lacked enzymatic activity (**Fig**. **3F**). Core efficiently co-immunoprecipitated with the catalytically inactive DGAT1 mutant indicating that core binding to DGAT1 occurs independently from DGAT1-mediated triglyceride synthesis and lipid droplet formation (**Fig**. **3G**).

**Figures 3A-G**. **A**. Co-immunoprecipitation assays in 293T cells co-transfected with expression vectors for core and FLAG-DGAT1 or FLAG-DGAT2. DGAT proteins were immunoprecipitated with α-FLAG agarose followed by western blotting with α-core and α-FLAG antibodies. **B.** Co-immunoprecipitation of core with endogenous DGAT1 in Huh7 cells transduced with core-expressing lentiviral vectors. DGAT1 was immunoprecipitated with α-DGAT1 antibodies bound to protein A agarose. **C.** Indirect immunofluorescence of core and endogenous DGAT1 in Huh7 cells transfected with wildtype or mutant (SPMT) core expression vectors (scale bar = 10 µm). **D.** Co-immunoprecipitation assays in 293T cells co-transfected with expression vectors for wildtype (WT), mutant (SPMT) or truncated (1-173) core together with FLAG-DGAT1. (e-f) 293T cells were transfected with FLAG-DGAT1, FLAG-DGAT1 (N389A), FLAG-DGAT1 (H426A), and FLAG-DGAT1 (N389A, H426A). **E.** Analysis of DGAT expression by western blotting. * marks an unspecific band that serves as loading control. Of note, overexpressed FLAG-DGAT1 sometimes displayed a double band in western blotting indicating that it might be posttranslationally modified. However, we never detected a double band for endogenous DGAT1. **F.** *In vitro* DGAT activity assay. Extracted lipids were loaded on a thin layer chromatography plate and analyzed by autoradiography. All assays were performed in triplicate. Bands in control lanes represent endogenous DGAT activity. **G.** Co-immunoprecipitation of core and FLAG-DGAT1 or catalytically inactive FLAG-DGAT1 (H426A) in 293T cells.

### Example 4: DGAT1 inhibition impairs HCV virion assembly

It was hypothesized that the induction of lipid droplets by HCV Core protein is important for the viral life-cycle. For these studies, an eGFP reporter virus was constructed that contains, in order from 5' to 3', the HCV 5'UTR, an enhanced green fluorescent protein (eGFP) reporter, a second internal ribosome entry site (IRES) from equine cytomegalovirus (ECMV), and the genes of the highly infectious, partially cell culture adapted strain Jc1. This reported virus is termed eGFP-Jc1 (Pietschmann, et al. 2006. Proc. Natl. Acad. Sci. USA 103:7408-7413).

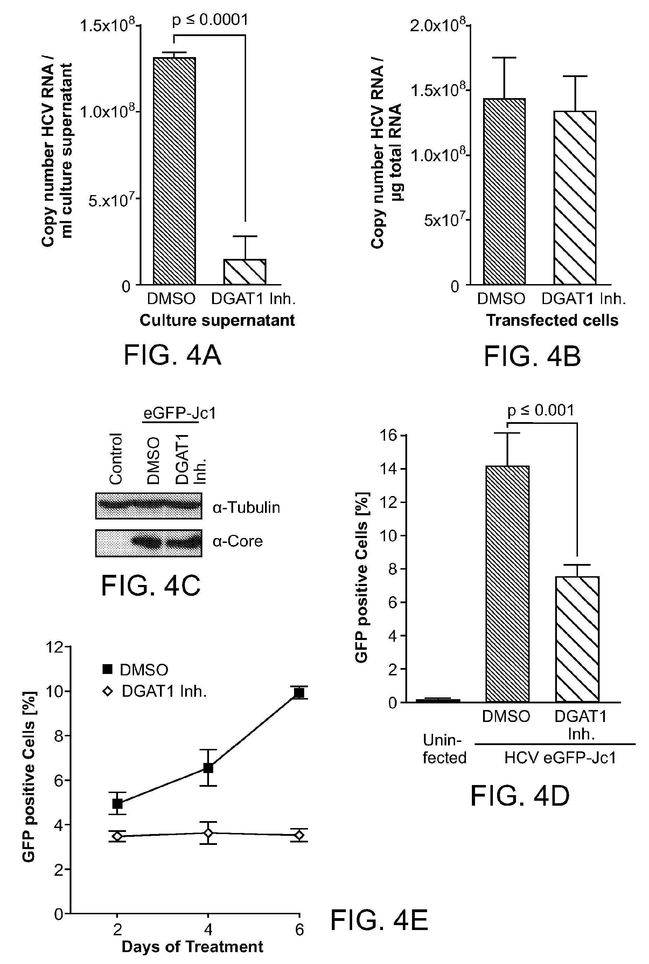

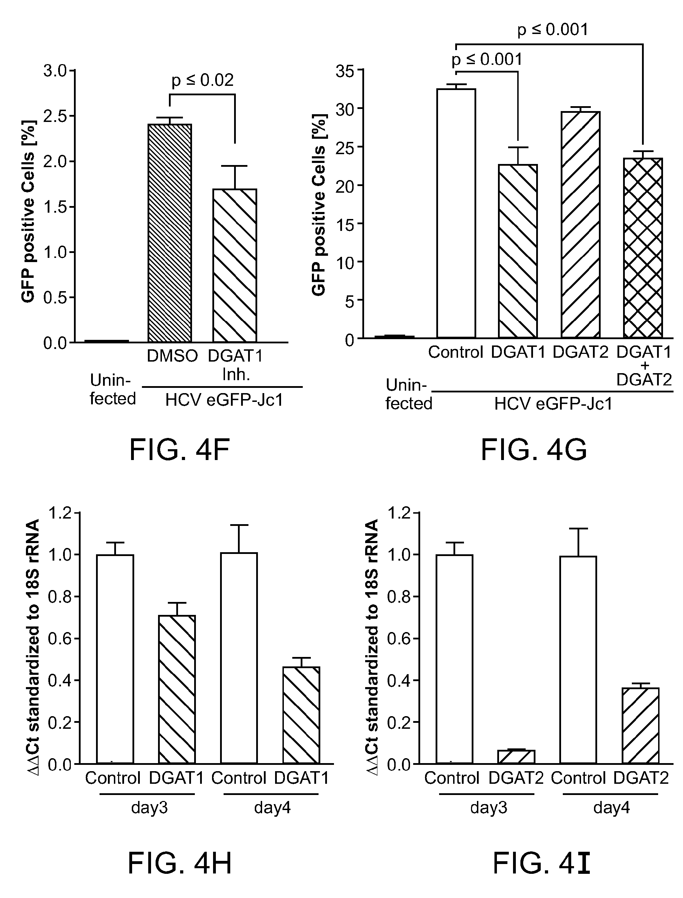

The impact of DGAT1 inhibition on the viral life-cycle was assessed. The release of HCV RNA in the culture supernatant of eGFP-Jc1-transfected cells treated with the DGAT1 inhibitor was measured by quantitative polymerase chain reaction (qPCR). As shown in **Figure 4A****,** DGAT1 inhibition reduces the amount of released HCV RNA more than 80% compared to control cells. To analyze whether DGAT1 inhibition affects viral RNA replication, total cellular RNA was isolated, and viral RNA was quantified by qPCR. DGAT1 inhibition does not inhibit HCV RNA replication and does not affect translation of the viral proteins as shown by immunoblot of the core protein **Figure 4B and 4C****.**

To confirm that the lower levels of HCV RNA in the culture supernatants reflect fewer infectious particles, the supernatant of treated cultures were used to infect naive cells, and the number of infected cells was measured by fluorescence activated cell sorting (FACS) analysis 2 days post infection. Infectivity of the supernatant of HCV transfected cells treated with the DGAT1 inhibitor was significantly reduced compared to control treated culture (**Figure 4D**). In a time-course experiment in which secreted virus was harvested on different days after the beginning of DGAT1 inhibitor treatment, it was found that in control cells, there is a steady increase in virus secretion, while in the DGAT1 inhibitor treated cultures, secretion levels of do not change over time. (**Figure 4E**).

It has been postulated that HCV exits the cell via the lipoprotein export machinery. Inhibition of the microsomal transfer protein or knock-down of either ApoB100 or ApoE resulted in marked decreased virus particle release but an accumulation of intracellular infectious particles. Inhibition of the low density lipoprotein (LDL) export machinery inhibits particle release without affecting the assembly of intracellular infectious particles (Chang, et al. (2007) J. Virol. 81:13783-13793; Huang et al. (2007) Proc. Natl. Acad. Sci. USA 104:5848-5853). In contrast, DGAT1 inhibition not only decreases virion release but also significantly reduces the amount of intracellular infectious particles. Therefore DGAT1 inhibition seems to affect the virus assembly step rather than blocking secretion of infectious particles.

To confirm the results obtained with the DGAT1 Inhibitor, siRNAs were used to knock-down DGAT1 and DGAT2. The following siRNA were used:
siRNA DGAT1: 5'-CUUGAGCAAUGCCCGGUUA-3' (SEQ ID NO:11); and
siRNA DGAT2: 5'-GAACACACCCAAGAAAGGU-3' (SEQ ID NO:15).

A -50% knock-down of DGAT1 and a ∼80% knock-down of DGAT2 in Huh7 cells 3-4 days post-transfection, as quantified by qPCR, was archived (**Figures 4H** **an**d **4I**). The effect of siRNAs on HCV was analyzed by quantifying spreading infection. Huh7.5 cells were transfected with siRNAs, infected with equal amounts of concentrated eGFP-Jc1 reporter virus on day 3 and analyzed 3 days later for spreading infection by measuring the amount of GFP positive cells by flow cytometry. DGAT1 knock-down significantly impaired spreading infection of the virus compared to non-targeting control siRNAs (**Figure 4G**). DGAT2 only has a minor effect on spreading infection and knock-down of both DGAT1 and DGAT2 is not additive (**Figure 4G**). These results suggest that HCV uniquely depends on DGAT1 activity to efficiently release viral particles.

**Figures 4A-I.** Huh7.5 cells were electroporated eGFP-Jc1 RNA and treated with DMSO or 20 µM DGAT1 inhibitor on day 1 post-transfection (p.t.). **A.** RNA was isolated from the culture supernatant on day 4 p.t. HCV RNA was quantified by RT-qPCR. Shown are mean, S.D. and p values for n = 6. **B.** Total RNA was isolated on day 4 p.t. HCV RNA was quantified by RT-qPCR, normalized to 18S rRNA and quantified via a standard. Shown are mean, S.D. and p values for n=6. **C.** Cells were lysed for sodium dodecyl sulfate-polyacrylamide gel electrophoresis (SDS-PAGE) (day 3). Immunoblot with α-Core and α-Tubulin antibodies. **D.** Culture supernatant was harvested (day 3 p.t.), filtered and concentrated. Naive Huh7.5 cells were infected with the virus and analyzed by flow cytometry 2 days post-infection (p.i.). Shown are mean and S.D. of one experiment (n=3). p values were calculated of the means of independent experiments (n=6). **E.** At the indicated days the culture supernatant was harvested, filtered and concentrated. Naive Huh7.5 cells were infected with the virus and analyzed by flow cytometry 2 days p.i. Shown are mean and S.D. of one representative experiment (n=3). **F.** Intracellular virus was obtained by 3 freeze thaw cycles. Naive Huh7.5 cells were infected and analyzed by flow cytometry 2 days p.i. Shown are mean and S.D. of one experiment (n=3). p values were calculated of the means of independent experiments (n=4). **G.** Huh7.5 cells were electroporated with siRNA (day 0) and infected with low amounts of concentrated eGFP-Jc1 virus for 3h on day 3. 6 days p.t. (3 days p.i.) cells were harvested and analyzed by flow cytometry for spreading infection. Shown are mean, S.D. and p values for n = 5. **H-I.** Huh7.5 cells were electroporated with siRNA. At the indicated time points total cellular RNA was isolated using RNA Stat reagent. DGAT1 (**H**) and DGAT2 (**I**) expression levels were obtained by RT-pRCR using DGAT1 and DGAT2 specific Taqman Probes via the deltadeltaCT method with 18S rRNA as an internal standard. Shown is 1 representative experiment.

### Example 5: Lack of DGAT1 suppresses HV spreading infection.

### Method

Small hairpin RNAs targeting DGAT1 (1393: GGAACATCCCTGTGCACAA (SEQ ID NO:16); 1417: GCATCAGACACTTCTACAA (SEQ ID NO:17)); DGAT2 (1812: GCGAAAGCCACTTCTCATA; SEQ ID NO:18); and luciferase control (CTTACGCTGAGTACTTCGA; SEQ ID NO:19) were cloned into a modified version of the pSicoR lentiviral vector that encodes a mCherry reporter driven by an EF-1α promoter (pSicoRMS)(Ventura et al. (2004) Proc. Natl. Acad. Sci. USA 101:10380; Grskovic et al. (2007) PLoS Genet. 3:e145). Lentiviral particles were produced as previously described (Naldini et al. (1996). Science 272:263-267). Briefly, 293T cells were cotransfected with the transfer plasmid encoding the pSicoRMS shRNA constructs, an HIV-based packaging construct (pCMVΔR8.91) and a construct expressing the glycoprotein of vesicular stomatitis virus (VSV-G) (pMD.G). Culture supernatant containing pseudotyped lentiviral particles was concentrated using ultracentrifugation for 16 h at 20,000 rpm in a SW28 rotor. Infectious titres were determined by transducing NIH/3T3 cells with serial dilutions of the viral stocks and FACS analysis 2 days post-transduction. Transductions were carried out in the presence of 4 µg/ml polybrene (Sigma) for 4 h at 37 °C.

### Results

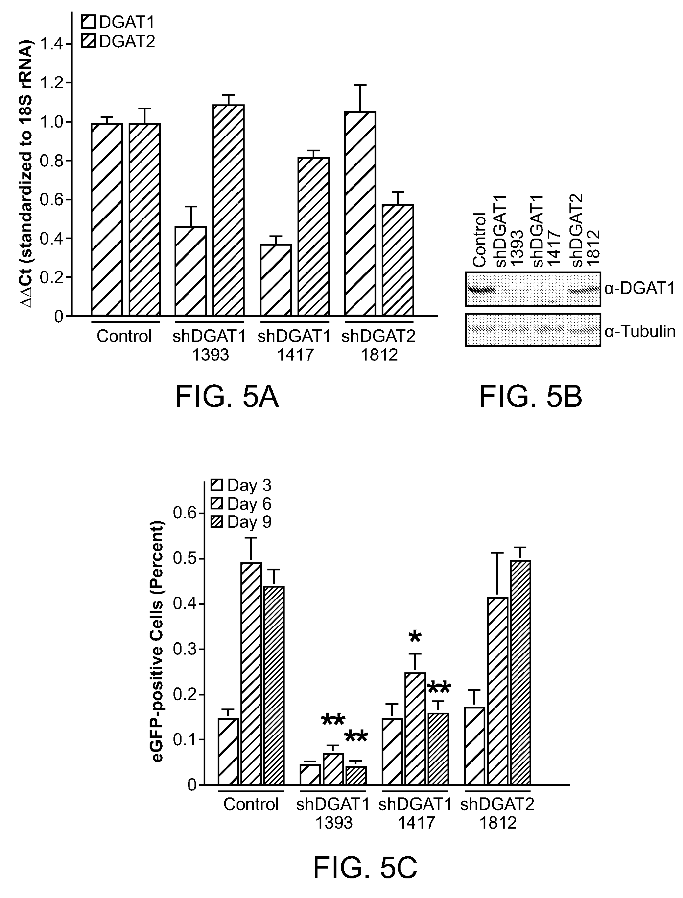

Short hairpin RNAs (shRNAs) directed against DGAT1 or DGAT2 were introduced by lentiviral vector transduction into a permissive subclone of the Huh7 hepatoma cell line (Huh7.5). Knockdown of DGAT expression was verified by real-time RT-PCR and, in the case of DGAT1, by western blotting (**Fig. 5A and 5B**). Knockdown cells were inoculated with low concentrations of an infectious HCV reporter virus (eGFP-Jc1), and viral spread was analyzed by flow cytometry of eGFP. Spreading infection was efficiently suppressed with two separate hairpins directed against DGAT1, while no change was induced with a hairpin specific for DGAT2 (**Fig. 5C**).

**Figures 5A-C**. **A-C**. Knockdown of DGAT1 or DGAT2 in Huh7.5 cells with lentiviral vectors expressing shRNAs directed against DGAT1 or DGAT2. Knockdown was evaluated by real-time RT-PCR from total cellular RNA (mean ± s.e.m.; n = 4) (A) or by western blot with a-DGAT1 antibodies (B). No antibody reliably detecting endogenous human DGAT2 enzyme is currently available. **C.** Knockdown Huh7.5 cells were inoculated with low concentrations of eGFP-Jc1 viral stock to measure viral spreading infection. Samples were analyzed by flow cytometry of eGFP on the indicated days post infection (mean ± s.e.m.; n = 7; *p < 0.05, **p < 0.01).

### Example 6: DGAT1 inhibition suppresses viral protein and RNA recruitment to lipid droplets.

### Method

Lipid droplets were isolated as described (Miyanari et al. (2007) Nat. Cell Biol. 9:1089). Briefly, cells were scraped in phosphate buffered saline (PBS), lysed in hypotonic buffer (50 mM HEPES, 1 mM EDTA and 2 mM MgCl₂, pH 7.4) supplemented with protease inhibitors with 30 strokes in a tight-fitting Dounce homogenizer. After spinning 5 min at 1500 rpm, post nuclear fractions were mixed with equal volumes of 1.05 M sucrose in isotonic buffer (50 mM HEPES, 100 mM KCl, 2 mM MgCl₂) and placed at the bottom of SW55 Ti (Beckman) centrifuge tubes, overlaid with isotonic buffer containing 1 mM phenylmethylsulphonyl fluoride (PMSF) and centrifuged for 2 h at 100,000 x g. Proteins from the floating lipid droplet fraction were precipitated with 15% trichloroacetic acid and 30% acetone, washed once with acetone and resuspended in urea loading dye (200 mM Tris/HCl pH 6.8, 8 M urea, 5% sodium dodecyl sulfate (SDS), 1 mM ethylenediaminetetraacetic acid (EDTA), 0.1 % bromophenol blue, 15 mM dithiothreitol (DTT)).

### Results

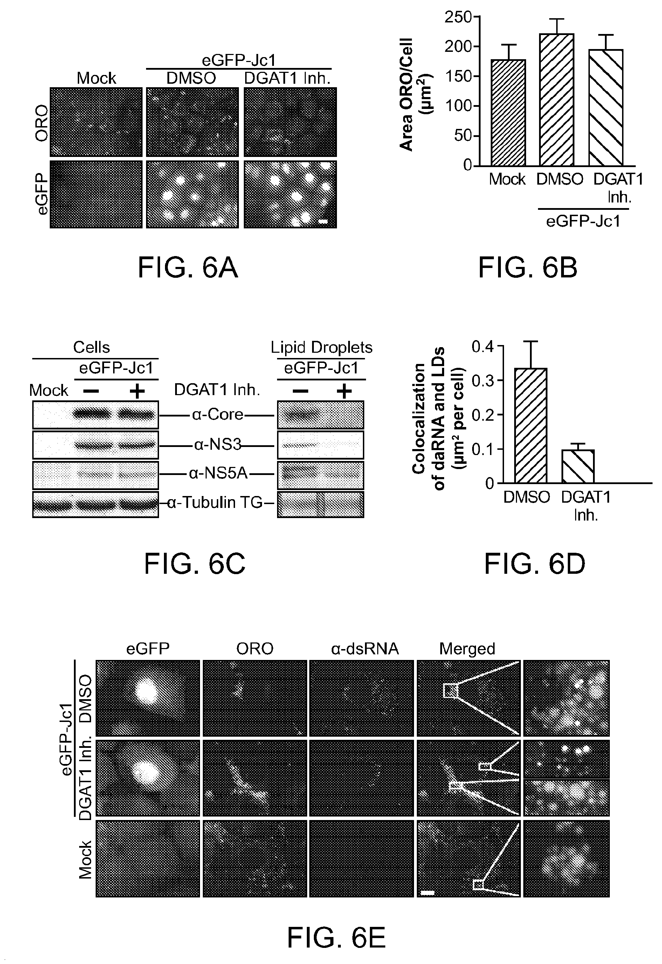

As treatment with the DGAT1 inhibitor did not change the overall lipid droplet content in infected hepatoma cells (**Figures 6A and 6B**), it was examined whether core binding to lipid droplets was affected. Lipid droplet fractions were isolated from eGFP-Jc1 transfected cells treated with the DGAT1 inhibitor or vehicle control. While core was readily detected in lipid droplet fractions from control-treated cells, no core was found at lipid droplets in cells treated with DGAT1 inhibitor (**Fig. 6C**). Intracellular core production was unaffected by the treatment consistent with the model that RNA replication and viral translation are not influenced by DGAT1 (**Fig. 6C**). Similar results were obtained for viral NS5A and NS3 proteins, which together with core localize to lipid droplets during active HCV particle production (**Fig**. **6C**)(Miyanari et al. (2007) *supra;* Tellinghuisen, et al. (2008) J. Virol. 82:1073; Ma et al. (2008) J. Virol. 82:7624-7639). Intracellular triglyceride content remained the same in the presence or absence of the DGAT1 inhibitor, as observed for intracellular lipid droplet content (Fig. 6C; TG).

A critical function of core at lipid droplets is the recruitment of viral RNA for encapsidation (Miyanari et al. (2007) *supra*). To analyze whether this process requires DGAT1, eGFP-Jc1-transfected cells were stained with antibodies specific for double-stranded RNA that reliably detect double-stranded HCV RNA (Targett-Adams et al. (2008) J. Virol. 82:2182). While in vehicle-treated cells a subset of lipid droplets was decorated with signals for double-stranded RNA, very little overlap was seen after DGAT1 inhibitor treatment (**Fig. 6D and 6E**). No signal at all was detected in mock-transfected hepatoma cells confirming that the antibodies specifically react with double-stranded HCV RNA (Fig. 6E; Mock).

**Figures 6A-C**. **A-E**. Huh Lunet cells were electroporated with *in vitro* transcribed eGFP-Jc1 RNA (day 0) and treated with dimethylsulfoxide (DMSO) or 20 µM DGAT1 inhibitor (day 1). Cells were fixed for indirect immunofluorescence or processed for lipid droplet isolation on day 3 post transfection. **A.** ORO staining. **B.** Quantification of (A) (mean of 1000 cells ± SEM). (scale bar 20 µm). **C.** Western blot analysis of cell extracts or isolated lipid droplet fractions. TG: extracted triglyceride analyzed by thin layer chromatography. **D.** Quantification of double-stranded RNAs localized at lipid droplets in cells described above (mean of 30 cells ± s.e.m.). **E.** Indirect immunofluorescence of double-stranded RNA at lipid droplets (bar = 10 µm).

The invention is further characterized by the following numbered embodiments:
1. A method of treating a hepatitis C virus infection in an individual, the method comprising administering to the individual an effective amount of an active agent that reduces the level and/or activity of a lipid synthesis acyltransferase.
2. The method of embodiment 1, wherein the lipid synthesis acyltransferase is a diacylglycerol acyltransferase-1 (DGAT1) polypeptide, wherein said DGAT1 polypeptide comprises an amino acid sequence having at least about 75% amino acid sequence identity to the amino acid sequence set forth in SEQ ID NO:1.
3. The method of embodiment 1, wherein the lipid synthesis acyltransferase is a diacylglycerol acyltransferase-1 (DGAT2) polypeptide, wherein said DGAT2 polypeptide comprises an amino acid sequence having at least about 75% amino acid sequence identity to the amino acid sequence set forth in SEQ ID NO:2.
4. The method of embodiment 1, wherein the lipid synthesis acyltransferase is an acyl-CoA: cholesterol acyltransferase-1 (ACAT1) polypeptide, wherein said ACAT1 polypeptide comprises an amino acid sequence having at least about 75% amino acid sequence identity to the amino acid sequence set forth in SEQ ID NO:3.
5. The method of embodiment 1, wherein the lipid synthesis acyltransferase is an acyl-CoA:cholesterol acyltransferase-2 (ACAT2) polypeptide, wherein said ACAT2 polypeptide comprises an amino acid sequence having at least about 75% amino acid sequence identity to the amino acid sequence set forth in SEQ ID NO:4.
6. The method of embodiment 1, wherein the active agent is a small molecule inhibitor of a lipid synthesis acyltransferase.
7. The method of embodiment 1, wherein the active agent is an interfering RNA that specifically reduces the level of a lipid synthesis acyltransferase in a cell.
8. The method of embodiment 1, wherein the active agent is an antibody that specifically binds a lipid synthesis acyltransferase.
9. The method of embodiment 1, wherein the active agent is administered in an amount effective to reduce HCV viral titers to fewer than about 5000 genome copies/mL serum.
10. The method of embodiment 1, wherein a sustained viral response is achieved.
11. The method of embodiment 1, wherein the method further comprises administering to the individual an effective amount of a nucleoside analog.
12. The method of embodiment 11, wherein the nucleoside analog is selected from ribavirin, levovirin, viramidine, an L-nucleoside, and isatoribine.
13. The method of embodiment 1, wherein the method further comprises administering to the individual an effective amount of an interferon-alpha (IFN-α).
14. The method of embodiment 13, wherein the IFN-α is monoPEG (30 kD, linear)-ylated consensus IFN-α.
15. The method of embodiment 13, wherein the IFN-α is INFERGEN consensus IFN-α.
16. The method of embodiment 13, wherein the IFN-α is PEGASYS™PEGylated IFN-α2a or PEG-INTRON™PEGylated IFN-a2b.
17. The method of embodiment 1, further comprising administering to the individual an NS3 protease inhibitor, an NS5B polymerase inhibitor, or an NS3 helicase inhibitor.
18. The method of embodiment 1, wherein the HCV is genotype 1b.
19. The method of embodiment 1, wherein said administering is by subcutaneous injection or intramuscular injection.
20. The method of embodiment 1, wherein said administering is by oral delivery.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims.

## Claims

1. An agent that reduces the level and/or activity of a lipid synthesis acyltransferase in an individual with liver steatosis for use in a method of treatment of liver steatosis in an individual.

2. An agent for use according to claim 1, wherein the lipid synthesis acyltransferase is a diacylglycerol acyltransferase-1 (DGAT1) polypeptide, wherein said DGAT1 polypeptide comprises an amino acid sequence having at least about 75% amino acid sequence identity to the amino acid sequence set forth in SEQ ID NO:1.

3. An agent for use according to claim 1, wherein the lipid synthesis acyltransferase is a diacylglycerol acyltransferase-1 (DGAT2) polypeptide, wherein said DGAT2 polypeptide comprises an amino acid sequence having at least about 75% amino acid sequence identity to the amino acid sequence set forth in SEQ ID NO:2.

4. An agent for use according to any one of claims 1-3, wherein the percent by weight of fat in the liver of the individual is at least 5%.

5. An agent for use according to any one of claims 1-3, wherein the active agent is administered in an amount effective to reduce the percent by weight of fat in the liver of the individual by at least 10%.

6. An agent for use according to claim 2, wherein the agent is a small molecule inhibitor of DGAT 1.

7. An agent for use according to claim 6, wherein the small molecule inhibitor of DGAT1 is 2-((1s,4s)-4-(4-(4-amino-7,7-dimethyl-7H-pyrimido[4,5-b][1,4]oxazin-6-yl)phenyl)cyclohexyl)acetic acid, or a derivative or analog thereof.

8. An agent for use according to claim 6, wherein the small molecule inhibitor of DGAT1 is an oxadiazole compound of the formula: 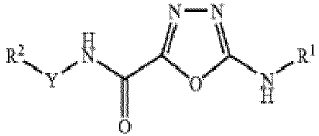
in which R¹ is an optionally substituted aryl or optionally substituted hetero aryl group;
Y is a direct bond, or a group (CR⁴⁰R⁴¹)ₛ or -X6(CR⁴⁰R⁴¹)ₜ, where each of R⁴⁰ and R⁴¹ is independently selected from hydrogen, (1-4C)alkyl, hydroxyl, halo, halo(1-4C)alkyl, amino, cyano, (1-4C)alkoxy, (1-4C)haloalkoxy or ((1-3)alkyl)CONH-, and where s is an integer of from 1 to 6 and t is an integer of from 1 to 6; and
R² is an optionally substituted aryl, an optionally substituted cycloalkyl or an optionally substituted heterocyclic group.

9. An agent for use according to claim 6, wherein the small molecule inhibitor of DGAT1 is a compound of the following formula: 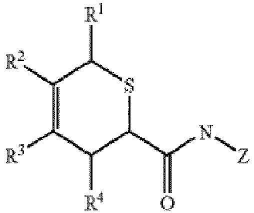
in which Z is selected from the group consisting of aryl and heteroaryl;
each aryl and heteroaryl may be optionally substituted with 1 to 3 R⁵;
R¹, R², R³, and R⁴ are independently selected from the group consisting of alkyl and alkoxy;
R³ and R⁴ may be taken together to from an aryl ring that is optionally substituted with 1 to 3 R⁶;
R⁵ is selected from the group consisting of alkyl, thioalkyl and halo; and
R⁶ is selected from the group consisting of alkyl and alkoxy.

10. An agent for use according to claim 6, wherein the small molecule inhibitor of DGAT1 is a compound of the following formula: 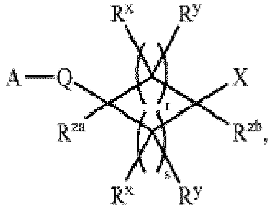
in which Q is a phenyl or a monocyclic heteroaryl;
A is phenyl, or a 4-, 5-, 6- or 7-memebered monocyclic ring selected from the group consisting of heteroaryl and heterocycle;
r and s are independently 1 or 2;
X is X¹, -(CR^{k}R^{m})ᵤ-X¹, -(CR^{k}R^{m})ᵤ-C(O) -X¹, or -C(O) -X¹, in which X¹ is heterocycle or heteroaryl;
q, t, u, v, and w, at each occurrence, are each independently 1, 2, 3, 4, 5, or 6; and
R^{x}, R^{y}, R^{za}, R^{zb}, R^{k} and R^{m} at each occurrence, are independently hydrogen, alkyl, or haloalkyl.

11. An agent for use according to claim 6, wherein the small molecule inhibitor of DGAT1 is a compound of the following formula: 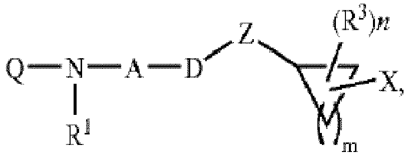
in which Q is -C(=Y)N(R²)(R^{2a}), -C(=W)(R^{b}), -R^{b}, -S(O)₂(R^{b}), or -C(O)O(R^{b});
R¹ and R^{2a} are each independently hydrogen or lower alkyl;
R² is alkyl, aryl, heteroaryl, cycloalkyl, cycloalkyenyl, or heterocycle;
R³ represents a substituent group selected from the group consisting of alkyl, haloalkyl, and halogen, m is 1, 2, 3, 4, or 5;
n is 0, 1, or 2;
A and D are each a monocyclic ring selected from the group consisting of phenyl, heteroaryl, cycloalkyl, and cycloalkenyl;
Z is C(O), C(H)(OH), C(alkyl)(OH), O, N(R^{b}), S(O), S(O)₂, or CH₂;
X represents a substituent group selected from the group consisting of -C(O)OR⁵, -C(O)N(R⁵)₂, -CN, -C(=NOR⁵)N(R⁵)₂, -C(R⁶R⁷)OH, -C(O) -N(R⁵)(OR⁵), and tetrozolyl;
R⁴, at each occurrence, is independently aryl, heteroaryl, cycloalkyl, cycloaklenyl, or heterocycle;
R⁵, at each occurrence, is independently hydrogen, alkyl, or haloalkyl;
R⁶ and R⁷ are independently hydrogen or alkyl, or R⁶ and R⁷ together with the carbon atom to which they are attached, form a three to six-membered, monocyclic ring selected from the group consisting of cycloalkyl and cycloalkenyl; and
R^{b}, at each occurrence, is independently alkyl, ahloalkyl, or R⁴.

12. An agent for use according to claim 6, wherein the small molecule inhibitor of DGAT1 is a compound of the following formula: 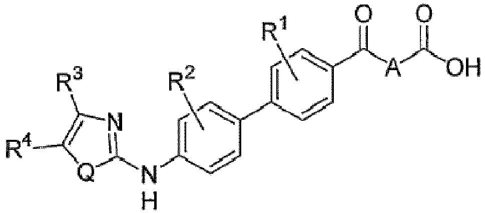
in which Q is O, S, or NR⁵;
A is a linker;
R¹ and R² are independently selected from hydrogen, halo, (C₁-C₆)alkyl, and (C₁-C₆)alkoxy; R³ is selected from hydrogen, (C₁-C₆)alkyl optionally substituted by hydroxy, and phenyl optionally substituted with (C₁-C₆)alkyl, (C₁-C₆)alkoxy, or halo. R⁴ is selected from hydrogen, nitro, and (C₁-C₆)alkyl; and
R⁵ is hydrogen or (C₁-C₆)alkyl.

13. An agent for use according to claim 3, wherein the active agent is a small molecule inhibitor of DGAT2.

14. An agent for use according to claim 13, wherein the DGAT2 inhibitor is a polymethoxylated flavone (PMF).

15. An agent for use according to claim 14, wherein the PMF is a citrus flavonoid.

16. An agent for use according to claim 13, wherein the DGAT2 inhibitor is vitamin B₃.
